# EUROPEAN PATENT SPECIFICATION

(11) **EP 2 347 696 B1**
(45) Date of publication and mention of the grant of the patent: **29.03.2017**
(21) Application number: 09821852.2
(22) Date of filing: 24.06.2009
(51) Int. Cl.: A61B 1/04, A61B 5/06, A61B 5/07, A61B 1/00

(54) **CAPSULE GUIDANCE SYSTEM**
KAPSELFÜHRUNGSSYSTEM
SYSTÈME DE GUIDAGE DE CAPSULE

(30) Priority: 21.10.2008 JP 2008271213
(43) Date of publication of application: 27.07.2011
(73) Proprietor: Olympus Corporation, Tokyo 192-8507 (JP); Siemens Healthcare GmbH, 91052 Erlangen (DE)
(72) Inventor: KIMURA, Atsushi, Tokyo 192-8507 (JP); TAKIZAWA, Hironobu, Tokyo 192-8507 (JP); KAWANO, Hironao, Tokyo 192-8507 (JP); KUTH, Rainer, 91315 Hoechstadt/Aisch (DE)
(74) Representative: Gunzelmann, Rainer
(86) International application number: PCT/JP2009/061526
(87) International publication number: WO 2010/047152

(56) References cited:
- WO-A1-2008/004497
- WO-A1-2008/155828
- JP-A- 2003 116 781
- JP-A- 2003 116 781
- JP-A- 2005 168 524
- JP-A- 2005 185 644
- US-A1- 2004 044 279
- US-A1- 2006 041 181
- US-A1- 2008 043 902
- US-A1- 2008 091 171

## Description

### Field

The present invention relates to a capsule guiding system for guiding a capsule medical device which is introduced into a subject such as a patient as defined in claim 1.

### Background

In the field of endoscopy, there has been a capsule medical device provided with an imaging function and a wireless communication function in a capsule-shaped casing which is formed in size capable of being introduced into an alimentary canal of a subject such as a patient. The capsule medical device is moved within the alimentary canal by peristalsis and the like after being swallowed through a mouth of the subject. The capsule medical device sequentially takes images at the inside of an internal organ of the subject (hereinafter, sometimes called in-vivo images) over a period of time from being introduced into the alimentary canal of the subject until being naturally excreted to the outside of the subject, and then, sequentially performs wireless transmission of the taken in-vivo images to a receiving device outside the subject.

Capsule guiding systems for guiding with magnetic force (i.e., magnetic guiding) a capsule medical device, which is introduced into a subject, have also been proposed. Generally, in a capsule guiding system, a capsule medical device provided with a magnet in a capsule-shaped casing is introduced into an organ of a subject. Then, the capsule medical device in the subject is magnetically guided by applying a magnetic field to the capsule medical device in the subject from the outside.

Such capsule guiding system includes, for example, a system in which a capsule medical device is magnetically guided while detecting a position and a direction of the capsule medical device in a subject (see Patent Literature 1 and Patent Literature 2) and a system in which a capsule medical device in a stopped state is magnetically guided as detecting whether or not the capsule medical device in a subject is in a stopped state at a stenosis part and the like in an organ (see Patent Literature 3). Further, there has been a system to record image data taken by a capsule medical device in a subject in association with the imaging position thereof (see Patent Literature 4).
Japanese Patent Application Publication Number JP 2003 116781 A describes a capsule type medical equipment capable of early detecting clogging by a stenosis. There is provided a capsule type endoscope to be inserted into a body cavity that images the inside of the body cavity and transmits the image to an external unit located outside the body by a radio wave from an antenna. The external unit receives the radio wave at an antenna unit composed of a plurality of antennas, calculates a position of the capsule type endoscope from a signal intensity utilizing a position calculation circuit, and calculates a change in position at predetermined times. The external unit determines a state where the capsule type endoscope is clogged within the stenosis when a positional change is below a threshold for a predefined time, and early detects a clogged state of the capsule type endoscope by displaying a message on a liquid crystal monitor and by sounding a buzzer.

### Citation List

### Patent Literature

Patent Literature 1: Japanese Laid-open Patent Publication No. 2007-216040
Patent Literature 2: International Publication No. 2005/112733
Patent Literature 3: US Patent No. 7076284
Patent Literature 4: US Patent Laid-open No. 2006/0202998

### Summary

### Technical Problem

Incidentally, an in-vivo image group taken by the capsule medical device in a subject is taken into an image display device via a receiving device outside the subject and displayed on a display unit of the image display device. A user such as a doctor or a nurse magnetically guides the capsule medical device in the subject by operating an operation unit of the capsule guiding system while observing the in-vivo image group displayed on the display unit. In this manner, the user observes the in-vivo images taken by the capsule medical device while moving the capsule medical device along an alimentary canal of the subject. Under such circumstances, a situation may take place in which it is desired to observe once more an in-vivo site through which the capsule medical device has already passed.

However, with the above-mentioned capsule guiding system in the related art, the user has to magnetically guide the capsule medical device in the subject by operating the operation unit similarly to the case of frontward moving of the capsule medical device in the subject when backwardly moving the capsule medical device in the subject by the magnetic guiding from the current position to a previously passed position (i.e., a past position relative to the current position). Accordingly, there has been a problem of large operational burden for a user to backwardly move the capsule medical device in a subject to a past position.

In particular, there may be a case that the capsule medical device in the subject is backwardly moved while directing an imaging field in a direction being different from the backward direction (for example, being the opposite direction) at the inside of a bowel such as a small intestine and a large intestine. In this case, since the user has to operate the magnetic guiding with the operation unit while observing the in-vivo images in a direction being different from the backward direction of the capsule medical device, the operational burden for the user is further increased.

The present invention has been made in view of the above situation, and an object of the present invention is to provide a capsule guiding system capable of relieving operational burden for a user when backwardly moving a capsule medical device in a subject.

### Solution to Problem

To solve the problem described above and achieve the object, a capsule guiding system according to the present invention is disclosed as defined in claim 1.

In the capsule guiding system according to the present invention as set forth in the invention described above, the input unit inputs mark information to be assigned to the in-vivo image displayed on the display unit, the storage unit sequentially stores guidance information of the capsule guiding unit for guiding the capsule medical device from a certain past position being an imaging position of the in-vivo image to which the mark information is assigned to a current position at which the capsule medical device currently exists, and the control unit controls the capsule guiding unit to guide the capsule medical device from the current position to the past position.

In the capsule guiding system according to the present invention as set forth in the invention described above, the storage unit sequentially stores an in-vivo image taken by the capsule medical device in association with guidance information of the capsule guiding unit, and the control unit controls the capsule guiding unit to guide the capsule medical device to an imaging position of a past in-vivo image relative to an imaging position of an in-vivo image currently displayed on the display unit.

In the capsule guiding system according to the present invention as set forth in the invention described above, the input unit inputs mark information to be assigned to the in-vivo image displayed on the display unit, the storage unit sequentially stores an in-vivo image taken by the capsule medical device in association with guidance information of the capsule guiding unit, and the control unit extracts, from the storage unit, guidance information of the capsule guiding unit for guiding the capsule medical device from a past position being an imaging position of the in-vivo image to which the mark information is assigned to a current position and controls the capsule guiding unit to guide the capsule medical device from the current position to the past position based on the extracted guidance information.

In the capsule guiding system according to the present invention as set forth in the invention described above, a position detection unit that detects position information of the capsule medical device at the inside of the subject is further included, the display unit displays trajectory information connecting the position information of the capsule medical device, the storage unit sequentially stores the position information of the capsule medical device in association with the guidance information of the capsule guiding unit, the input unit inputs designation information to designate past position information of the capsule medical device included in the trajectory information, and the control unit controls the capsule guiding unit to guide the capsule medical device from a current position to a past position corresponding to the past position information.

In the capsule guiding system according to the present invention as set forth in the invention described above, the control unit sequentially calculates distance difference between position information of the capsule medical device to be guided toward the past position and the trajectory information and controls the capsule guiding unit to stop guiding of the capsule medical device when the distance difference exceeds a predetermined threshold value.

In the capsule guiding system according to the present invention as set forth in the invention described above, the control unit determines whether or not the capsule medical device has arrived at a vicinity of the past position being targeted and controls the capsule guiding unit to reduce guiding speed of the capsule medical device to low speed after arriving at the vicinity of the past position compared to that before arriving thereat.

In the capsule guiding system according to the present invention as set forth in the invention described above, the control unit determines whether or not the capsule medical device has arrived at the past position being targeted and controls the capsule guiding unit to keep the capsule medical device staying at the arrived past if the capsule medical device has arrived at the past position.

In the capsule guiding system according to the present invention as set forth in the invention described above, the control unit determines whether or not the capsule medical device has arrived at the past position being targeted and controls the capsule guiding unit to swing the capsule medical device if the capsule medical device has arrived at the past position.

In the capsule guiding system according to the present invention as set forth in the invention described above, the input unit inputs frontward-moving instruction information to instruct frontward moving of the capsule medical device, and the control unit controls the capsule guiding unit to cause the capsule medical device to move frontward on a trajectory based on the guidance information of the capsule guiding unit stored in the storage unit when the frontward instruction information is input.

In the capsule guiding system according to the present invention as set forth in the invention described above, the control unit extracts at least one or more characteristic images having predetermined character from among an in-vivo image group of the subject taken by the capsule medical device and controls the display unit to display the extracted characteristic images, and the input unit inputs the mark information to be assigned to any of the characteristic images displayed on the display unit.

In the capsule guiding system according to the present invention as set forth in the invention described above, the input unit further inputs additional information to be added to the in-vivo image displayed on the display unit, and the storage unit stores the in-vivo image, the additional information and the guidance information of the capsule guiding unit in association with each other.

In the capsule guiding system according to the present invention as set forth in the invention described above, the mark information is information related to attractive force exerted on the capsule medical device by the capsule guiding unit.

In the capsule guiding system according to the present invention as set forth in the invention described above, the mark information is information related to torque exerted on the capsule medical device by the capsule guiding unit.

In the capsule guiding system according to the present invention as set forth in the invention described above, the mark information is information related to movement speed of the capsule medical device.

In the capsule guiding system according to the present invention as set forth in the invention described above, the mark information is information related to a previously-defined variation pattern of a magnetic field to be generated by the capsule guiding unit.

### Advantageous Effects of Invention

With the capsule guiding system according to the present invention, the capsule medical device takes an in-vivo image of a subject as being introduced into the subject, the capsule guiding unit guides the capsule medical device, the image acquisition unit acquires the in-vivo image of the subject from the capsule medical device, the display unit displays the in-vivo image of the subject acquired by the image acquisition unit, the storage unit sequentially stores guidance information of the capsule guiding unit that guides the capsule medical device, the input unit inputs instruction information to instruct backward moving of the capsule medical device, and the control unit controls, when the instruction information is input, the capsule guiding unit such that the capsule medical device backwardly moves on a trajectory based on the guidance information of the capsule guiding unit stored in the storage unit. Accordingly, it is not necessary for a user such as a doctor or a nurse to manually operate backward guiding of the capsule medical device and the capsule medical device in a subject can be backwardly moved to a desired in-vivo position automatically at desired timing. As a result, there arises an effect to actualize the capsule guiding system capable of relieving operational burden for a user to backwardly move the capsule medical device in a subject.

### Brief Description of Drawings

FIG. 1 is a schematic view illustrating a structural example of a capsule guiding system according to a first embodiment of the present invention.
FIG. 2 is a schematic view illustrating a structural example of a capsule medical device in the capsule guiding system according to the first embodiment of the present invention.
FIG. 3 is a flowchart exemplifying operation procedure of the capsule guiding system when the capsule medical device is backwardly guided in a subject.
FIG. 4 is a schematic view exemplifying a state that the capsule medical device backwardly moves on an own movement trajectory in a bowel.
FIG. 5 is a schematic view schematically illustrating a structural example of a capsule guiding system according to a second embodiment of the present invention.
FIG. 6 is a flowchart exemplifying operation procedure of the capsule guiding system when the capsule medical device is backwardly guided to a target position in a subject.
FIG. 7 is a flowchart describing an example of operation procedure of a control unit of the capsule guiding system according to the second embodiment until a target position setting process is achieved.
FIG. 8 is a schematic view illustrating an example of a state that the capsule medical device in a small intestine backwardly moves to the target position on an own movement trajectory.
FIG. 9 is a schematic view schematically illustrating a structural example of a capsule guiding system according to a third embodiment of the present invention.
FIG. 10 is a flowchart describing an example of operation procedure of a control unit of the capsule guiding system according to the third embodiment until a target position setting process is achieved.
FIG. 11 is a schematic view illustrating an example of a state that the capsule medical device backwardly moves on an own movement trajectory to a target position being an imaging position of an in-vivo image to which a mark information is assigned.
FIG. 12 is a schematic view schematically illustrating a structural example of a capsule guiding system according to a fourth embodiment of the present invention.
FIG. 13 is a flowchart describing an example of operation procedure of the capsule guiding system to backwardly guide the capsule medical device to a target position while controlling backward guiding speed.
FIG. 14 is a schematic view illustrating an example of a state that the capsule medical device is backwardly guided as slowing the backward guiding speed in the vicinity of a target position.
FIG. 15 is schematic view schematically illustrating a structural example of a capsule guiding system according to a fifth embodiment of the present invention.
FIG. 16 is a flowchart describing an example of operation procedure of the capsule guiding system to keep the capsule medical device, which is backwardly guided to a target position, in a staying state.
FIG. 17 is a schematic view illustrating an example of a state that the capsule medical device is kept staying at the target position.
FIG. 18 is a schematic view schematically illustrating a structural example of a capsule guiding system according to a sixth embodiment of the present invention.
FIG. 19 is a flowchart describing an example of operation procedure of the capsule guiding system to swing the capsule medical device with magnetic force.
FIG. 20 is a schematic view illustrating an example of a state that the capsule medical device is swung at a target position in a subject.
FIG. 21 is a schematic view schematically illustrating a structural example of a capsule guiding system according to a seventh embodiment of the present invention.
FIG. 22 is a flowchart describing an example of operation procedure until a control unit of the capsule guiding system according to the seventh embodiment achieves a target position setting process.
FIG. 23 is a schematic view illustrating an example of a state that the capsule medical device is backwardly guided to the target position being an imaging position of a characteristic image to which mark information is assigned.

### Description of Embodiments

Hereinafter, embodiments of a capsule guiding system according to the present invention will be described in detail with reference to the drawings. In the following, a capsule guiding system that magnetically guides a capsule medical device (e.g., a capsule endoscope) which takes in-vivo images of a subject is described as an example. However, the embodiments do not limit the present invention.

### First Embodiment

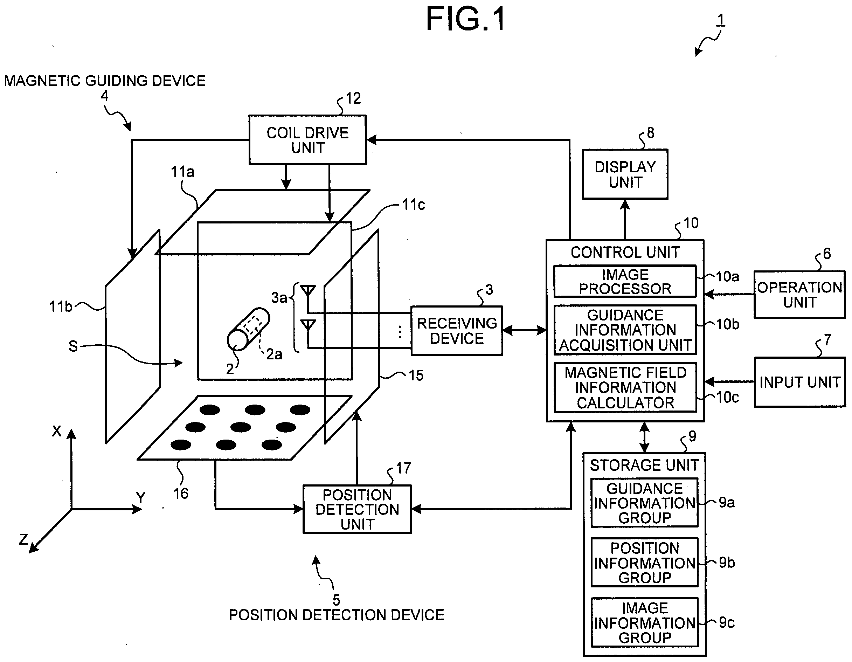

FIG. 1 is a schematic view illustrating a structural example of a capsule guiding system according to a first embodiment of the present invention. As illustrated in FIG. 1, a capsule guiding system 1 according to the first embodiment includes a capsule medical device 2 to take in-vivo images of a subject, a receiving device 3 to receive an image signal from the capsule medical device 2, a magnetic guiding device 4 to magnetically guide the capsule medical device 2 introduced into the subject, and a position detection device 5 to detect position information of the capsule medical device 2 at the inside of the subject. Further, the capsule guiding system 1 includes an operation unit 6 to operate magnetic guiding of the capsule medical device 2 with the magnetic guiding device 4, an input unit 7 to input various types of information, a display unit 8 to display various types of information such as in-vivo images of the subject, a storage unit 9 to store various types of information, and a control unit 10 to control the respective structural units of the capsule guiding system 1.

The capsule medical device 2 is a capsule-type medical device to acquire in-vivo information of the subject such as in-vivo images and is provided with an imaging function and a wireless communication function in a capsule-shaped casing. The capsule medical device 2 is introduced into an organ of the subject such as a patient and is moved within the organ of the subject thereafter, and finally, excreted to the outside of the subject. The capsule medical device 2 sequentially takes in-vivo images of the subject at predetermined intervals over a period of time from being introduced into the inside of the organ of the subject until being excreted to the outside of the subject, and sequentially transmits wireless signals including the taken in-vivo images to the receiving device 3 outside the subject. Further, the capsule medical device 2 incorporates a magnetic body, such as a permanent magnet, or an electric magnet (hereinafter, simply called a magnet), and an LC marker 2a which is an LC resonance circuit utilizing a coil and a capacitor. After being introduced into the subject, the capsule medical device 2 is magnetically guided by the magnetic guiding device 4 while the position detection device 5 detects a position of the capsule medical device 2 at the inside of the subject. Incidentally, not being limited to the LC method utilizing the LC marker 2a, the position detection method for the capsule medical device 2 with the position detection device 5 may be another method. For example, the position detection device 5 may detect the position of the capsule medical device 2 at the inside of the subject based on intensity of a receiving electric field of a wireless signal from the capsule medical device 2 in the subject.

The receiving device 3 functions as an image acquisition unit to acquire in-vivo information of the subject from the capsule medical device 2 in the subject. Specifically, the receiving device 3 includes plural receiving antennas 3a. The plural receiving antennas 3a are discretely arranged on the body surface of the subject and capture wireless signals transmitted by the capsule medical device 2 in the subject. The receiving device 3 receives the wireless signals from the capsule medical device 2 in the subject via the plural receiving antennas 3a. The receiving device 3 performs a demodulation process etc. on the wireless signals from the capsule medical device 2 and acquires an in-vivo image of the subject included in the wireless signal. The receiving device 3 transmits an image signal of the acquired in-vivo image to the control unit 10.

The magnetic guiding device 4 functions as a capsule guiding unit to magnetically guide the capsule medical device 2 introduced into the subject. Specifically, the magnetic guiding device 4 includes magnetic field generation units 11a to 11c to generate a magnetic field for guidance to magnetically guide the capsule medical device 2 in the subject, and a coil drive unit 12 to supply electric current required for generating the magnetic field for guidance to the magnetic field generation units 11a to 11c. Here, the magnetic guiding device 4 of the simplest structure having the three magnetic field generation units 11a to 11c is exemplified in the following. However, though not illustrated, the magnetic guiding device 4 of the capsule guiding system 1 according to the present invention may be a device with plural (for example, four or more) magnetic field generation units to generate the magnetic field for guidance for the capsule medical device 2 by the plural magnetic field generation units.

The magnetic field generation units 11a to 11c are embodied by utilizing a coil and the like and generate the magnetic field for guidance to magnetically guide the capsule medical device 2 in the subject. Specifically, the magnetic field generation unit 11a generates a magnetic field in the X-axis direction of a three-axis orthogonal coordinate system which defines three-dimensional space S. The magnetic field generation unit 11b generates a magnetic field in the Y-axis direction of the three-axis orthogonal coordinate system. The magnetic field generation unit 11c generates a magnetic field in the Z-axis direction of the three-axis orthogonal coordinate system. Here, the subject in a state that the capsule medical device 2 is introduced into an organ is placed in the three-dimensional space S of which space coordinates are defined by the three-axis orthogonal coordinate system. The magnetic field generation units 11a to 11c generate the magnetic field for guidance to magnetically guide the capsule medical device 2 in the subject at the inside of the three-dimensional space S in which the subject exists. The magnetic field for guidance is formed by the magnetic field in the X-axis direction due to the magnetic field generation unit 11a, the magnetic field in the Y-axis direction due to the magnetic field generation unit 11b, or the magnetic field in the Z-axis direction due to the magnetic field generation unit 11c, or is formed by appropriately combining the respective magnetic fields due to the magnetic field generation units 11a to 11c. The magnetic field generation units 11a to 11c apply the magnetic field for guidance to the capsule medical device 2 in the subject, thereby magnetically guiding the capsule medical device 2 in the subject.

The coil drive unit 12 drives the magnetic field generation units 11a to 11c so as to generate the magnetic field for guidance. Specifically, the coil drive unit 12 appropriately generates an electric current signal for the magnetic field generation units 11a to 11c based on the control of the control unit 10. The coil drive unit 12 amplifies the generated electric current signal and appropriately inputs the amplified electric current signal respectively to the magnetic field generation units 11a to 11c. In this manner, the coil drive unit 12 supplies electric current required for generating the magnetic field for guidance to the magnetic field generation units 11a to 11c.

The position detection device 5 is for detecting a position of the capsule medical device in the subject. Specifically, the position detection device 5 includes: a magnetic field generation unit 15 to generate a magnetic field for detection to be applied to the LC marker 2a of the capsule medical device 2; a magnetic field detection unit 16 to detect an induced magnetic field generated from the LC marker 2a as a result of an effect of the magnetic field for detection; and a position detection unit 17 to detect the position of the capsule medical device 2 at the inside of the subject based on the detection result of the induced magnetic field of the magnetic field detection unit 16.

The magnetic field generation unit 15 is embodied by utilizing a coil and the like and generates the magnetic field for detection to detect the position of the capsule medical device 2 in the subject. Specifically, the magnetic field generation unit 15 generates the magnetic field for detection in the three-dimensional space S in which the subject exists and applies the magnetic field for detection to the LC marker 2a of the capsule medical device 2 in the subject. Accordingly, the magnetic field generation unit 15 causes the LC marker 2a to generate the induced magnetic field.

The magnetic field detection unit 16 detects the induced magnetic field generated from the LC marker 2a of the capsule medical device 2. Specifically, the magnetic field detection unit 16 includes plural coils for magnetic field detection (e.g., nine coils for magnetic field detection in FIG. 1) and detects the induced magnetic field from the LC marker 2a with the plural coils for magnetic field detection. The magnetic field detection unit 16 converts the detected induced magnetic field from the LC marker 2a into a voltage signal for each coil for magnetic field detection and transmits the voltage signal of each coil for magnetic field detection to the position detection unit 17 as the detection result of the induced magnetic field from the LC marker 2a. Here, the number of the coils for magnetic field detection arranged in the magnetic field detection unit 16 is not limited to nine as long as being plural. Further, the plural coils for magnetic field detection in the magnetic field detection unit 16 may be arranged in a matrix state or in another arrangement state.

The position detection unit 17 detects the position of the capsule medical device 2 in the subject by utilizing the magnetic field generation unit 15 and the magnetic field detection unit 16. Specifically, based on the control of the control unit 10, the position detection unit 17 generates an electric current signal for the magnetic field generation unit 15 and amplifies the generated electric current signal. The position detection unit 17 inputs the amplified electric current signal to the magnetic field generation unit 15, thereby supplying electric current required for generating the magnetic field for detection to the magnetic field generation unit 15. Further, the position detection unit 17 sequentially acquires a voltage signal being a detection result of the induced magnetic field from the LC marker 2a as described above from the magnetic field detection unit 16. The position detection unit 17 detects position information of the capsule medical device 2 in the subject based on the detection result of the induced magnetic field of the LC marker 2a acquired from the magnetic field detection unit 16. In this case, the position detection unit 17 calculates the position coordinates and a direction vector of the capsule medical device 2 in the three-dimensional space S. The position detection unit 17 transmits the position information of the capsule medical device 2 in the subject to the control unit 10. Here, the position information includes each of information of the position coordinates and the direction vector of the capsule medical device 2 calculated by the position detection unit 17.

The operation unit 6 is a unit to operate to magnetically guide the capsule medical device 2 with the magnetic guiding device 4. Specifically, the operation unit 6 is embodied by utilizing an input device such as a joystick and an input button. The operation unit 6 inputs instruction information to the control unit 10 to instruct to magnetically guide the capsule medical device 2 in response to input operation of a user such as a doctor or a nurse.

The input unit 7 is embodied by utilizing an input device such as a keyboard and a mouse, and inputs various types of information to the control unit 10 in response to input operation of a user such as a doctor or a nurse. Specifically, the input unit 7 inputs instruction information to instruct to the control unit 10. More specifically, the input unit 7 inputs instruction information to instruct backward moving of the capsule medical device 2 in the subject, instruction information to instruct displaying of an in-vivo image of the subject taken by the capsule medical device 2, instruction information to instruct displaying of trajectory information of the capsule medical device 2 in the subject, and the like. Further, the input unit 7 inputs patient information to identify the subject, such as a patient name of the subject, a patient ID, birth date, sex and age, examination information to identify examination for the subject such as an examination ID and examination date, and the like.

The display unit 8 is embodied by utilizing any of various displays such as a CRT display or a liquid crystal display and displays various types of information which is instructed to be displayed by the control unit 10. Specifically, the display unit 8 displays an in-vivo image group of the subject taken by the capsule medical device 2, the patient information and the examination information of the subject, the position information of the capsule medical device 2 in the subject, the trajectory information indicating the movement trajectory of the capsule medical device 2 in the subject, and the like.

The storage unit 9 is embodied by utilizing various types of storage media in which rewritable information is stored, such as a RAM, an EEPROM, a flash memory or a hard disk. The storage unit 9 stores various types of information which is instructed by the control unit 10 to be stored and transmits, to the control unit 10, the information which is instructed by the control unit 10 to be read out among the various types of stored information. Specifically, the storage unit 9 sequentially stores the guidance information of the magnetic guiding device 4 to magnetically guide the capsule medical device 2 in the subject as a guidance information group 9a, sequentially stores the position information of the capsule medical device 2 in the subject as a position information group 9b, and sequentially stores the in-vivo images of the inside of the subject taken by the capsule medical device 2 as an image information group 9c. Further, the storage unit 9 stores various types of input information input by the input unit 7.

The control unit 10 controls operation of the magnetic guiding device 4, the position detection device 5, the operation unit 6, the input unit 7, the display unit 8 and the storage unit 9 which are structural components of the capsule guiding system 1 and controls input and output of signals between the respective structural components. Specifically, the control unit 10 controls magnetic guiding of the capsule medical device 2 with the magnetic guiding device 4 based on the instruction information input through the operation unit 6. In this case, the control unit 10 controls the coil drive unit 12 based on the instruction information from the operation unit 6 to control supply quantity of.electric current for the magnetic field generation units 11a to 11c, thereby controlling the intensity and direction of the magnetic field for guidance in order to magnetically guide the capsule medical device 2 in the subject. Further, when the instruction information to instruct backward moving of the capsule medical device 2 in the subject is input through the input unit 7, the control unit 10 controls the magnetic guiding device 4 so that the capsule medical device 2 backwardly moves on the own movement trajectory in the subject based on the one or more types of guidance information included in the guidance information group 9a in the storage unit 9. In this case, the control unit 10 controls the intensity and direction of the magnetic field for guidance through the control of the supply quantity of electric current for the magnetic field generation units 11a to 11c, thereby controlling magnetic guiding of the capsule medical device 2 in the subject to backwardly move as tracing the own movement trajectory (hereinafter, called backward guiding).

Further, the control unit 10 causes the display unit 8 to display various types of information such as the in-vivo image group of the subject and the trajectory information of the capsule medical device 2, causes the storage unit 9 to store various types of information such as the guidance information group 9a, the position information group 9b and the image information group 9c, and causes the storage unit 9 to read out the stored information, based on the instruction information input by the input unit 7. Further, the control unit 10 controls start and stop of operation of the receiving device 3 and controls start and stop of operation of the position detection unit 17.

Meanwhile, the control unit 10 includes an image processor 10a to generate the in-vivo images of the subject, a guidance information acquisition unit 10b to acquire guidance information of the magnetic guiding device 4, and a magnetic field information calculator 10c to calculate magnetic field information for performing backward guiding of the capsule medical device 2. The image processor 10a sequentially acquires image signals from the receiving device 3 and generates the in-vivo image (i.e., the in-vivo image taken by the capsule medical device 2) of the subject in each time by performing a predetermined imaging process on the acquired image signal. The control unit 10 causes the display unit 8 to sequentially display the in-vivo images generated by the image processor 10a. Further, the control unit 10 causes the storage unit 9 to sequentially store the in-vivo images generated by the image processor 10a as a part of the image information group 9c.

The guidance information acquisition unit 10b acquires the guidance information of the magnetic guiding device 4 when the capsule medical device 2 is magnetically guided each time when the magnetic guiding device 4 magnetically guides the capsule medical device 2 in the subject. Specifically, the guidance information acquisition unit 10b calculates the intensity and direction of the magnetic field for guidance applied to the capsule medical device 2 based on the electric current signal which is generated at the coil drive unit 12 by the control unit 10 when the capsule medical device 2 is magnetically guided, that is, based on the supply quantity of electric current from the coil drive unit 12 to the magnetic field generation units 11a to 11c at the time of magnetic guiding of the capsule medical device 2. The guidance information acquisition unit 10b acquires the magnetic field information of the magnetic field for guidance as the guidance information of the magnetic guiding device 4. Here, the magnetic field information of the magnetic field for guidance includes magnetic field intensity information and magnetic field direction information of the magnetic field for guidance. The guidance information acquisition unit 10b acquires all of the guidance information of the magnetic guiding device 4 in chronological order when the capsule medical device 2 is magnetically guided during the capsule medical device 2 exists in the subject. The control unit 10 sequentially stores the guidance information acquired by the guidance information acquisition unit 10b in the storage unit 9 as a part of the guidance information group 9a.

The magnetic field information calculator 10c calculates the magnetic field information when the magnetic guiding device 4 performs the backward guiding of the capsule medical device 2. Specifically, the magnetic field information calculator 10c calculates the magnetic field information of the magnetic field for guidance required for the backward guiding of the capsule medical device 2 based on the guidance information group 9a accumulated in the storage unit 9. More specifically, the magnetic field information calculator 10c sequentially reads out each of guidance information included in the guidance information group 9a in reverse chronological order from the latest and sequentially calculates the magnetic field intensity information and the magnetic field direction information of the magnetic field for guidance for the backward guiding for each of the read guidance information. In this case, the magnetic field information calculator 10c calculates magnetic field direction information in the direction opposite to the magnetic field direction information in the guidance information and magnetic field intensity information being the same as the magnetic field intensity information in the guidance information, based on the read guidance information, for example. The control unit 10 controls the supply quantity of electric current to the magnetic field generation units 11a to 11c based on the magnetic field information calculated by the magnetic field information calculator 10c, thereby controlling the magnetic guiding device 4 to perform backward guiding of the capsule medical device 2 as applying the magnetic field for guidance having the magnetic field intensity and the magnetic field direction, which correspond to the magnetic field information, to the capsule medical device 2.

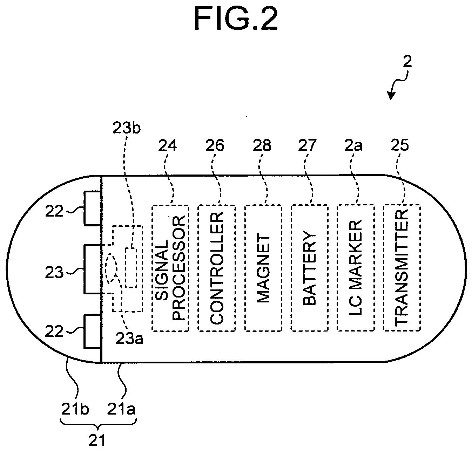

Subsequently, the configuration of the capsule medical device 2 will be described in detail. FIG. 2 is a schematic view illustrating a structural example of the capsule medical device in the capsule guiding system according to the first embodiment of the present invention. As illustrated in FIG. 2, the capsule medical device 2 according to the first embodiment includes a capsule-shaped casing 21 formed in size capable of being introduced into an organ of the subject. The capsule-shaped casing 21 is formed in a way that one end (i.e., an open end) of a cylindrical casing 21a of which other end is dome-shaped is closed with a dome-shaped casing 21b. The cylindrical casing 21a is a casing which is non-transparent against light in a predetermined waveband such as visible light. The dome-shaped casing 21b is a dome-shaped optical member and is transparent against light in the predetermined waveband such as visible light.

The capsule-shaped casing 21 accommodates the LC marker 2a, an illumination unit 22, an imaging unit 23, a signal processor 24, a transmitter 25, a controller 26, a battery 27 and a magnet 28 in a liquid-tight state. In this case, the illumination unit 22 and the imaging unit 23 are arranged on the side of the dome-shaped casing 21b, and then, the LC marker 2a, the signal processor 24, the transmitter 25, the controller 26, the battery 27 and the magnet 28 are arranged on the side of the cylindrical casing 21a.

The illumination unit 22 is embodied by utilizing a light emitting element such as an LED and illuminates an imaging field of the imaging unit 23. The imaging unit 23 includes a condenser lens 23a and a solid-state imaging element 23b. The condenser lens 23a focuses a reflected light from an object illuminated by the illumination unit 22 on a receiving surface of the solid-state imaging element 23b to form an optical image of the object. The solid-state imaging element 23b receives the reflected light from the object focused by the condenser lens 23a and takes an image of the object, that is, an in-vivo image of the subject.

The signal processor 24 generates an image signal of the in-vivo image based on an output signal from the solid-state imaging element 23b. The transmitter 25 performs a predetermined modulating process and the like on the image signal, generates a wireless signal which includes the in-vivo image, and transmits the generated wireless signal to the outside (specifically, to the receiving device 3). The controller 26 controls each operation of the illumination unit 22, the imaging unit 23, the signal processor 24 and the transmitter 25. The battery 27 supplies drive power to the illumination unit 22, the imaging unit 23, the signal processor 24, the transmitter 25 and the controller 26.

The LC marker 2a being an LC resonance circuit which is formed by utilizing a coil and a capacitor is arranged at the inside of the capsule-shaped casing 21 in a state that a predetermined direction of the capsule-shaped casing 21 (for example, the longitudinal axis direction, the radial direction or the like of the capsule-shaped casing 21) and an axis direction of the coil are matched. The LC marker 2a having a predetermined resonance frequency emits an induced magnetic field as receiving the magnetic field for detection generated by the magnetic field generation unit 15 of the position detection device 5. The induced magnetic field from the LC marker 2a is detected by the magnetic field detection unit 16 of the position detection device 5.

The magnet 28 is arranged at the inside of the capsule-shaped casing 21 in a state that a predetermined direction of the capsule-shaped casing 21 (for example, the radial direction of the capsule-shaped casing 21) and the magnetization direction thereof are matched. The magnet 28 moves as following the magnetic field for guidance applied by the magnetic field generation units 11a to 11c of the magnetic guiding device 4, thereby actualizing rotation movement or displacement movement of the capsule-shaped casing 21 in a state of following the magnetic field for guidance. The capsule medical device 2 incorporating the magnet 28 is capable of being magnetically guided in the desired rotation direction, frontward direction or backward direction owing to the effect of the magnetic field for guidance of the magnetic guiding device 4.

The capsule medical device 2 having the above-mentioned structure is introduced into an alimentary canal of the subject with oral ingestion and the like and sequentially performs imaging and transmitting of the in-vivo images in the alimentary canal. The capsule guiding system 1 sequentially detects the position information of the capsule medical device 2 in the subject with the position detection device 5 and displays the acquired position information on the display unit 8 as the current position of the capsule medical device 2. In this case, the control unit 10 is capable of generating the trajectory information indicating the movement trajectory of the capsule medical device 2 as connecting the position information group of the capsule medical device 2 in chronological order and displaying the generated trajectory information on the display unit 8. Further, the control unit 10 sequentially displays the in-vivo image group taken by the capsule medical device 2 in chronological order on the display unit 8.

Meanwhile, a user (i.e., an operator) such as a doctor or a nurse operates magnetic guiding of the capsule medical device 2 in the subject by utilizing the operation unit 6 while observing the current position of the capsule medical device 2 in the subject as referring to the in-vivo image, the trajectory information or the like displayed on the display unit 8. In response to the manual operation of the operation unit 6 by the user, the magnetic guiding device 4 applies the magnetic field for guidance to the capsule medical device 2 in the subject and magnetically guides the capsule medical device 2 owing to the magnetic field for guidance. Here, the capsule guiding system 1 backwardly guides the capsule medical device 2 in the subject along the alimentary canal at desired timing as in a situation that the user desires to observe once again a past position through which the capsule medical device 2 in the subject has already passed. The display unit 8 sequentially displays the in-vivo image group taken while the capsule medical device 2 backwardly moves on the own movement trajectory. The user can observe once again the past position in the subject by observing the in-vivo image group displayed on the display unit 8 as described above.

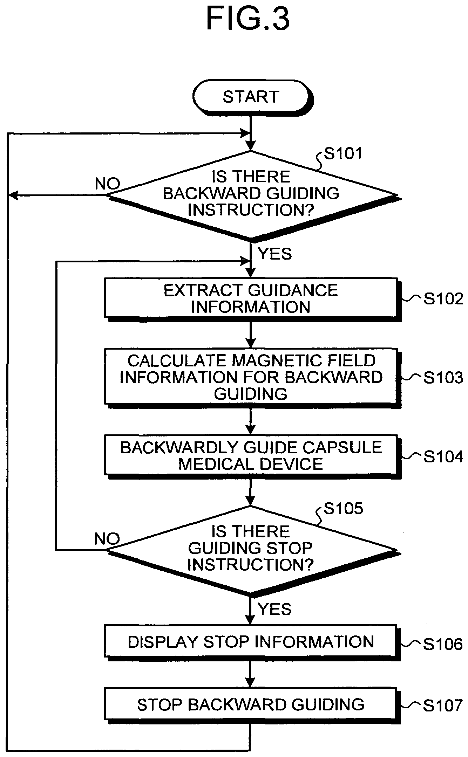

Subsequently, the operation of the capsule guiding system 1 when the capsule medical device 2 in the subject is backwardly guided will be described. FIG. 3 is a flowchart exemplifying operation procedure of the capsule guiding system when the capsule medical device in the subject is backwardly guided.

In the capsule guiding system 1 when the capsule medical device 2 in the subject is backwardly guided, the control unit 10 firstly determines whether or not there is an instruction of backward guiding, as indicated in FIG. 3 (step S101). In step S101, the control unit 10 observes presence or absence of input of backward moving instruction information to instruct backward guiding of the capsule medical device 2 in the subject. The control unit 10 determines that there is no backward guiding instruction ("No" in step S101) when the backward moving instruction information is not input. Step S101 is repeated until the backward moving instruction information is input. In this state, the control unit 10 is capable of controlling the magnetic guiding of the capsule medical device 2 in the subject with the magnetic guiding device 4 in response to the manual operation of the operation unit 6 by the user.

The user performs input operation (for example, depression of a button) of the backward moving instruction information by utilizing the input unit 7 at desired timing as in a situation that the user desires to observe once again a past position through which the capsule medical device 2 in the subject has already passed. The input unit 7 inputs the backward moving instruction information to the control unit 10 in response to the input operation by the user.

The control unit 10 determines that there is a backward guiding instruction ("Yes" in step S101) when the backward moving instruction information is input by the input unit 7 in step S101. Then, the control unit 10 extracts guidance information necessary for the backward guiding of the capsule medical device 2 in the subject from among the guidance information group 9a in the storage unit 9 (step S102). In step S102, the control unit 10 sequentially extracts the guidance information from among the guidance information group 9a in reverse chronological order.

Next, the control unit 10 calculates the magnetic field information for backwardly guiding the capsule medical device 2 in the subject (step S103). In step S103, the magnetic field information calculator 10c calculates the magnetic field intensity information and the magnetic field direction information of the magnetic field for guidance required for backwardly guiding the capsule medical device 2 based on the guidance information extracted from among the guidance information group 9a in step S102.

Subsequently, the control unit 10 controls the backward guiding of the capsule medical device 2 in the subject based on the magnetic field information calculated in step S103 (step S104). In step S104, through controlling of supply quantity of electric current for the magnetic field generation units 11a to 11c, the control unit 10 controls the magnetic guiding device 4 to apply the magnetic field for guidance to the capsule medical device 2 having the magnetic field intensity and the magnetic field direction corresponding to the magnetic field information calculated in step S103 as described above. In this case, the magnetic guiding device 4 backwardly guides the capsule medical device 2 in the subject as generating the magnetic field for guidance having the magnetic field intensity and the magnetic field direction by the process of step S103 based on the control of the control unit 10.

Then, the control unit 10 determines whether or not there is an instruction to stop guiding (step S105). In step S105, the control unit 10 observes presence or absence of input of stop instruction information to instruct stopping of backward guiding of the capsule medical device 2. The control unit 10 determines that there is no instruction to stop guiding ("No" in step S105) when the stop instruction information is not input. The procedure of step S102 and thereafter is repeated as returning to step S102. That is, the control unit 10 controls the magnetic guiding device 4 to continue the backward guiding of the capsule medical device 2 until the stop instruction information is input by the input unit 7.

The user performs input operation (for example, depression of a button) of the stop instruction information by utilizing the input unit 7 at desired timing such as the timing when backward moving of the capsule medical device 2 in the subject to the desired past position is completed. The input unit 7 inputs the stop instruction information to the control unit 10 in response to the input operation by the user.

The control unit 10 determines that there is an instruction to stop guiding ("Yes" in step S105) when the stop instruction information is input by the input unit 7 in step S105. Then, the control unit 10 controls the display unit 8 to display the stop information indicating that the backward guiding of the capsule medical device 2 is to be stopped (step S106). Subsequently, the control unit 10 controls the magnetic guiding device 4 to stop the backward guiding of the capsule medical device 2 (step S107). Then, the procedure of step S101 and thereafter is repeated as returning to step S101.

In step S107, the magnetic guiding device 4 stops applying of the magnetic field for guidance to the capsule medical device 2 in the subject based on the control of the control unit 10, thereby stopping the backward guiding of the capsule medical device 2. At that time, the capsule medical device 2 in the subject is in a state of being backwardly moved to the past position on the own movement trajectory, that is, a previously passed in-vivo position. Meanwhile, the user recognizes that the backward guiding of the capsule medical device 2 is stopped by observing the stop information displayed on the display unit 8.

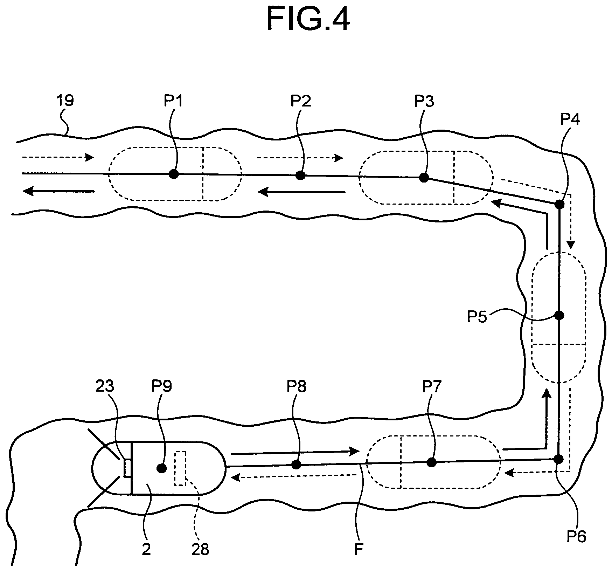

In the following, the operation of the capsule guiding system 1 according to the first embodiment is specifically described with an example in which the capsule medical device 2 located at the inside of a bowel (e.g., a small intestine and the like) of the subject is backwardly guided. FIG. 4 is a schematic view exemplifying a state that the capsule medical device in the bowel backwardly moves on the own movement trajectory. In the following, the operation of the capsule guiding system 1 will be described with reference to FIGS. 1 and 4.

The magnetic guiding device 4 magnetically guides the capsule medical device 2 frontward by applying the magnetic field for guidance to the magnet 28 of the capsule medical device 2 in the subject. The capsule medical device 2 in a state of being magnetically guided by the magnetic guiding device 4 sequentially moves frontward along the inside of an alimentary canal of the subject. For example, the capsule medical device 2 sequentially moves frontward along the small intestine 19 of the subject as indicated by dotted line arrows in FIG. 4. The capsule medical device 2 arrives at the current position P9 after sequentially passing through positions P1 to P8 at the inside of the small intestine 19 in a state that the imaging field of the imaging unit 23 is directed frontward.

In the capsule guiding system 1 which magnetically guides the capsule medical device 2 as described above, the guidance information acquisition unit 10b sequentially acquires, in chronological order, each of guidance information of the magnetic guiding device 4 which has magnetically guided the capsule medical device 2 until the capsule medical device 2 arrives at the position P9 after sequentially passing through the positions P1 to P8 in the direction of the dotted line arrows as indicated in FIG. 4. The control unit 10 sequentially stores each of the guidance information of the magnetic guiding device 4 in the storage unit 9 as a part of the guidance information group 9a.

Meanwhile, the position detection unit 17 sequentially detects the positions P1 to P9 of the capsule medical device 2 at the inside of the small intestine 19 in chronological order and sequentially transmits each of the position information of the positions P1 to P9 to the control unit 10. The control unit 10 causes the storage unit 9 to store each of the position information acquired from the position detection unit 17 as a part of the position information group 9b. Further, the control unit 10 generates the trajectory information indicating the movement trajectory F of the capsule medical device 2 as connecting each of the position information in chronological order and causes the display unit 8 to display the generated trajectory information. Here, the trajectory information of the movement trajectory F displayed on the display unit 8 is acquired by connecting each of point information corresponding to the positions P1 to P9 in FIG. 4 in chronological order with straight lines and the like.

Further, the control unit 10 acquires the in-vivo image group sequentially taken by the capsule medical device 2 until arriving at the position P9 from the receiving device 3 and causes the display unit 8 to sequentially display the acquired in-vivo image group in chronological order. The control unit 10 stores the in-vivo image group in the storage unit 9 as a part of the image information group 9c.

The user performs input operation of the backward moving instruction information by utilizing the input unit 7 when a past position on the movement trajectory F of the capsule medical device 2 at the inside of the small intestine 19 is desired to be observed once more. When the backward moving instruction information is input by the input unit 7, the control unit 10 controls the magnetic guiding device 4 so that the capsule medical device 2 backwardly moves on the movement trajectory F from the current position P9.

Specifically, the control unit 10 firstly extracts guidance information corresponding to the magnetic guiding from the position P8 to the position P9 out of the guidance information group 9a in the storage unit 9. The magnetic field information calculator 10c calculates the magnetic field intensity and the magnetic field direction of the magnetic field for guidance in order to backwardly guide the capsule medical device 2 from the position P9 to the position P8 based on the guidance information from the position P8 to the position P9. The control unit 10 controls the magnetic guiding device 4 so that the magnetic field for guidance having the calculated magnetic field intensity and magnetic field direction is to be generated. The magnetic guiding device 4 applies the magnetic field for guidance for the backward guiding to the capsule medical device 2 based on the control of the control unit 10, thereby backwardly guiding the capsule medical device 2 from the position P9 to the position P8.

Next, the control unit 10 extracts the guidance information corresponding to the magnetic guiding from the position P7 to the position P8 out of the guidance information group 9a in the storage unit 9. The magnetic field information calculator 10c calculates the magnetic field intensity and the magnetic field direction of the magnetic field for guidance in order to backwardly guide the capsule medical device 2 from the position P8 to the position P7 based on the guidance information from the position P7 to the position P8. The control unit 10 controls the magnetic guiding device 4 so that the magnetic field for guidance having the calculated magnetic field intensity and magnetic field direction is to be generated. The magnetic guiding device 4 applies the magnetic field for guidance for the backward guiding to the capsule medical device 2 based on the control of the control unit 10, thereby backwardly guiding the capsule medical device 2 from the position P8 to the position P7.

Subsequently, the control unit 10 repeats the processes similarly to the above case of backwardly guiding the capsule medical device 2 from the position P9 to the position P7 via the position P8 also for the past positions (for example, the positions P1 to P6 in FIG. 4) relative to the position P7 to cause the magnetic guiding device 4 to sequentially generate the magnetic field for guidance required for the backward guiding of the capsule medical device 2. The magnetic guiding device 4 backwardly guides the capsule medical device 2 so that the capsule medical device 2 backwardly moves on the movement trajectory F based on the control of the control unit 10. The control unit 10 continues to control the backward guiding of the capsule medical device 2 with the magnetic guiding device 4 until the stop instruction information of the backward guiding is input by the input unit 7.

The capsule medical device 2 backwardly guided by the magnetic guiding device 4 as described above backwardly moves on the movement trajectory F from the current position P9 as indicated by solid line arrows in FIG. 4. In this case, the capsule medical device 2 backwardly moves on the movement trajectory F in a state that the imaging field of the imaging unit 23 is directed to a direction being different from the backward direction, that is, to the opposite direction, to sequentially pass through the positions P8, P7, P6, P5, P4, P3, P2 and P1 in FIG. 4 in this order. The capsule medical device 2 continues the backward moving operation until the control unit 10 acquires the stop instruction information from the input unit 7.

As described above, in the first embodiment of the present invention, a capsule medical device in a subject is magnetically guided by a magnetic guiding device and guidance information of the magnetic guiding device when the capsule medical device is magnetically guided is sequentially stored in a storage unit. When instruction information for backward guiding of the capsule medical device is input by an input unit, a control unit controls the backward guiding of the capsule medical device with the magnetic guiding device based on the guidance information in the storage unit so that the capsule medical device backwardly moves on the own movement trajectory. Accordingly, it is not necessary for a user such as a doctor or a nurse to manually operate the backward guiding of the capsule medical device by utilizing an operation unit and the capsule medical device in the subject can be backwardly guided automatically at desired timing. As a result, it is possible to actualize a capsule guiding system in which operational burden for a user to backwardly move the capsule medical device in the subject by the magnetic guiding can be relieved.

A user can easily cause the capsule medical device 2 in the subject to backwardly move to a desired past position at desired timing by utilizing the capsule guiding system of the present invention. Accordingly, the user can easily observe once more the past in-vivo position through which the capsule medical device in the subject has already passed.

Further, in the first embodiment of the present invention, magnetic field information of a magnetic field for guidance for the backward guiding of the capsule medical device is calculated based on the guidance information of the magnetic guiding device which has actually magnetically guided the capsule medical device in the subject. Accordingly, it is possible to generate the magnetic field for guidance for the backward guiding having magnetic field intensity and a magnetic field direction in consideration of a situation of an alimentary canal through which the capsule medical device has passed by the magnetic guiding such as friction between the capsule medical device and an inner wall portion of the alimentary canal at the time of the magnetic guiding and a shape of the alimentary canal which is the movement path for the capsule medical device. As a result, the backward guiding of the capsule medical device can be reliably performed in accordance with the situation of the alimentary canal.

### Second Embodiment

In the following, a second embodiment of the present invention will be described. In the above first embodiment, the capsule medical device 2 in the subject is backwardly guided over a period from inputting of the backward moving instruction information until inputting of the stop instruction information of the backward guiding to the control unit 10. In the second embodiment, a target position for the backward guiding is set and the capsule medical device 2 is backwardly guided until arriving at the set target position.

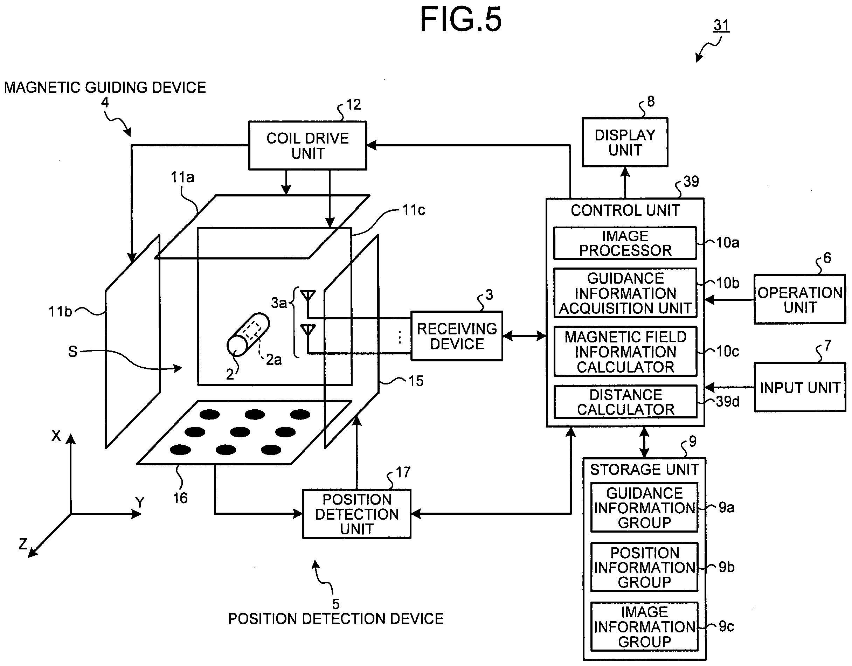

FIG. 5 is a schematic view schematically illustrating a structural example of a capsule guiding system according to the second embodiment of the present invention. As illustrated in FIG. 5, a capsule guiding system 31 according to the second embodiment is provided with a control unit 39 instead of the control unit 10 of the capsule guiding system 1 according to the above first embodiment. The rest of the structure is the same as the first embodiment and the same reference numeral is given to the same structural part.

The control unit 39 controls the storage unit 9 to store each of guidance information of the magnetic guiding device 4 sequentially acquired in chronological order by the guidance information acquisition unit 10b in association with each of position information of the capsule medical device 2 sequentially detected in chronological order by the position detection unit 17. That is, each of guidance information included in the guidance information group 9a and each of position information included in the position information group 9b in the storage unit 9 are respectively associated in chronological order by the control unit 39. When designation information of a target position is input by the input unit 7, the control unit 39 extracts the position information corresponding to the input designation information from among the position information group 9b and sets the extracted position information as the target position of the capsule medical device 2 in the subject. Here, the input unit 7 selectively inputs the designation information of the target position to the control unit 39 in response to predetermined input operation, such as clicking while locating a cursor at desired position information among respective position information (i.e., respective point information on the movement trajectory of the capsule medical device 2) included in the trajectory information of the capsule medical device 2 displayed on the display unit 8.

Further, the control unit 39 includes a distance calculator 39d in addition to the image processor 10a, the guidance information acquisition unit 10b and the magnetic field information calculator 10c. The distance calculator 39d appropriately calculates remaining distance from the current position of the capsule medical device 2 to the target position during the backward guiding and deviation distance between the current position of the capsule medical device 2 and the own movement trajectory during the backward guiding. Here, the deviation distance is the difference of distance between the position information and the trajectory information of the capsule medical device 2. The distance calculator 39d sequentially calculates the remaining distance and the deviation distance each time when acquiring the position information of the capsule medical device 2 from the position detection unit 17 during the backward guiding. Here, the remaining distance to the target position is remaining backward moving distance until the capsule medical device 2 arrives at the target position, and approximately equal to length of remaining movement trajectory from the current position to the target position of the capsule medical device 2 during the backward guiding. When the remaining distance calculated by the distance calculator 39d is equal to or smaller than a predetermined threshold value or the deviation distance calculated by the distance calculator 39d exceeds a predetermined threshold value, the control unit 39 controls the magnetic guiding device 4 to stop the backward guiding of the capsule medical device 2. Here, other functions of the control unit 39 are similar to those of the control unit 10 in the capsule guiding system 1 according to the above first embodiment.

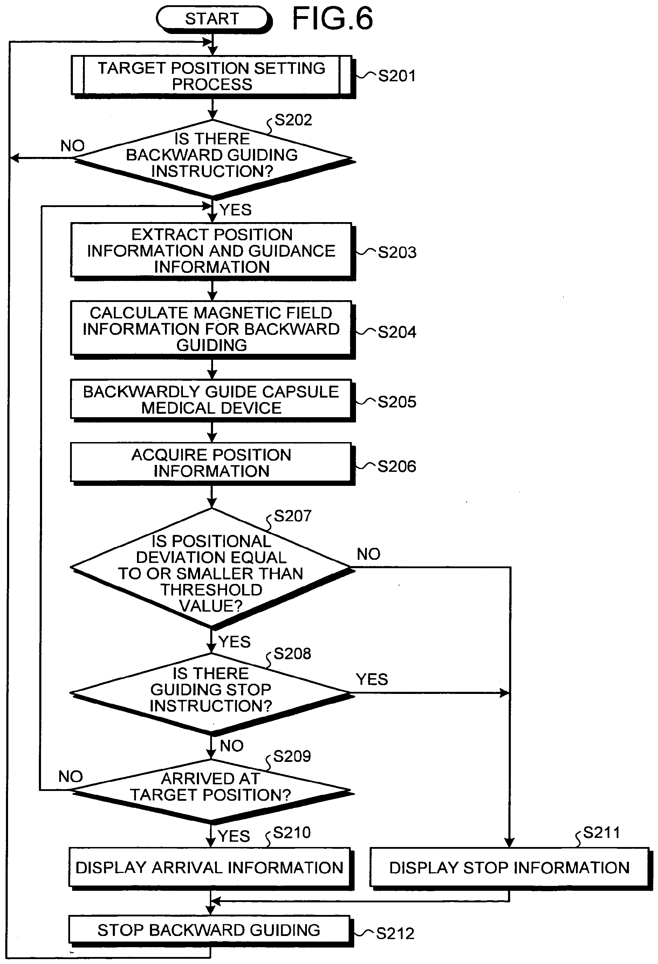

Next, the operation of the capsule guiding system 31 to backwardly guide the capsule medical device 2 in the subject will be described. FIG. 6 is a flowchart exemplifying operation procedure of the capsule guiding system to backwardly guide the capsule medical device in the subject to the target position.

In the capsule guiding system 31 when the capsule medical device 2 in the subject is backwardly guided, the control unit 39 firstly performs a target position setting process to set the target position of the capsule medical device 2 for the backward guiding (step S201), as indicated in FIG. 6. Then, similarly to step S101 as indicated in FIG. 3, it is determined whether or not there is an instruction of the backward guiding (step S202). When it is determined that there is no backward guiding instruction in step S202 ("No" in step S202), as returning to step S201, the control unit 39 repeats operation procedure of step S201 and step S202 until the backward moving instruction information is input by the input unit 7. In this state, the control unit 39 is capable of controlling magnetic guiding of the capsule medical device 2 in the subject with the magnetic guiding device 4 in response to manual operation of the operation unit 6 by a user.

On the contrary, when it is determined that there is a backward guiding instruction ("Yes" in step S202), the control unit 39 extracts, from the storage unit 9, the position information of the movement trajectory of the capsule medical device 2 in the subject and the guidance information corresponding to the position information (step S203). In step S203, the control unit 39 sequentially extracts the position information to form the own movement trajectory on which the capsule medical device 2 backwardly moves with the backward guiding (i.e., the movement trajectory of the capsule medical device 2 from the current position to the target position) from among the position information group 9b in the storage unit 9 in reverse chronological order. Further, the control unit 39 sequentially extracts the guidance information which is associated with the position information extracted from the position information group 9b from among the guidance information group 9a in the storage unit 9 in reverse chronological order.

Next, similarly to step S103 and step S104 in FIG. 3, the control unit 39 calculates the magnetic field information of the magnetic field for guidance required for backwardly guiding the capsule medical device 2 based on the guidance information extracted in step S203 (step S204). Then, the control unit 39 controls the backward guiding of the capsule medical device 2 in the subject based on the magnetic field information calculated in step S204, that is, the magnetic field intensity information and the magnetic field direction information of the magnetic field for guidance (step S205).

Subsequently, the control unit 39 acquires the position information of the capsule medical device 2 backwardly guided in step S205 (step S206). In step S206, the position detection unit 17 detects the current position information of the backwardly-guided capsule medical device 2. The control unit 39 acquires the current position information of the capsule medical device 2 for the backward guiding from the position detection unit 17.

Then, the control unit 39 determines whether or not positional deviation between the current position of the backwardly-guided capsule medical device 2 and the own movement trajectory is equal to or smaller than a predetermined threshold value (step S207). In step S207, the distance calculator 39d calculates the deviation distance between the current position of the capsule medical device in the backward guiding and the own movement trajectory on which the capsule medical device 2 is to backwardly move based on the current position information acquired from the position detection unit 17 in step S206 and the position information extracted in step S203. The control unit 39 performs a comparison process between the deviation distance calculated by the distance calculator 39d and the predetermined threshold value, and then, the control unit 39 determines whether or not the positional deviation between the current position of the capsule medical device 2 and the own movement trajectory is equal to or smaller than the predetermined threshold value based on the comparison process result.

When the deviation distance from the movement trajectory is equal to or smaller than the threshold value, the control unit 39 determines that the position deviation between the current position and the own movement trajectory of the capsule medical device 2 is equal to or smaller than the threshold value ("Yes" in step S207), and then, the control unit 39 determines whether or not there is an instruction to stop guiding (step S208) similarly to step S105 in FIG. 3. When it is determined that there is no instruction to stop guiding in step S208 ("No" in step S208), the control unit 39 determines whether or not the capsule medical device 2 has arrived at the target position (step S209).

In step S209, the distance calculator 39d calculates the remaining distance from the current position to the target position of the capsule medical device 2 for the backward guiding based on the current position information acquired from the position detection unit 17 in step S206 and each of position information to the target position set in step S201. The control unit 39 performs a comparison process between the remaining distance calculated by the distance calculator 39d and the predetermined threshold value, and then, the control unit 39 determines whether or not the capsule medical device 2 has arrived at the target position set by the target position setting process in step S201 based on the comparison process result.

When the remaining distance to the target position exceeds the threshold value, the control unit 39 determines that the capsule medical device 2 has not arrived yet at the target position ("No" in step S209). Then, the operation procedure of step S203 and thereafter is repeated as returning to step S203. On the contrary, when the remaining distance to the target position is equal to or smaller than the threshold value, the control unit 39 determines that the capsule medical device 2 has arrived at the target position ("Yes" in step S209). Then, the control unit 39 controls the display unit 8 to display arrival information which indicates arrival of the capsule medical device 2 at the target position (step S210).

Subsequently, similarly to step S107 in FIG. 3, the control unit 39 controls the magnetic guiding device 4 to stop the backward guiding of the capsule medical device 2 (step S212). Then, the operation procedure of step S201 and thereafter is repeated as returning to step S201. At that time, the capsule medical device 2 in the subject is in a state of having backwardly moved to the target position set in the target position setting process of step S201, that is, the desired in-vivo position on the movement trajectory of the capsule medical device 2. Meanwhile, the user recognizes that the capsule medical device 2 has backwardly moved to the desired target position by observing the arrival information displayed on the display unit 8.

On the contrary, when the deviation distance from the movement trajectory exceeds the threshold value in step S207, the control unit 39 determines that the positional deviation between the current position of the capsule medical device 2 and the own movement trajectory exceeds the threshold value. When it is determined that the positional deviation between the current position of the capsule medical device 2 and the own movement trajectory exceeds the threshold value in step S207 ("No" in step S207) or when it is determined that there is an instruction to stop guiding in step S208 ("Yes" in step S208), the control unit 39 controls the display unit 8 to display stop information which indicates stopping of the backward guiding of the capsule medical device 2 (step S211).

In step S211, when the capsule medical device 2 creates positional deviation exceeding the predetermined threshold value from the movement trajectory in the backward guiding, the control unit 39 causes the display unit 8 to display the stop information including warning information to warn of occurrence of the positional deviation. When stop instruction information of the backward guiding is input, the control unit 39 causes the display unit 8 to display the stop information which indicates stopping of the backward guiding of the capsule medical device 2 based on the stop instruction information.

The control unit 39 which has performed the operation procedure of step S211 repeats the operation procedure of step S212 and thereafter as proceeding to step S212. At that time, the user recognizes that the capsule medical device 2 has stopped the backward moving operation without arriving at the desired target position by observing the stop information displayed on the display unit 8.

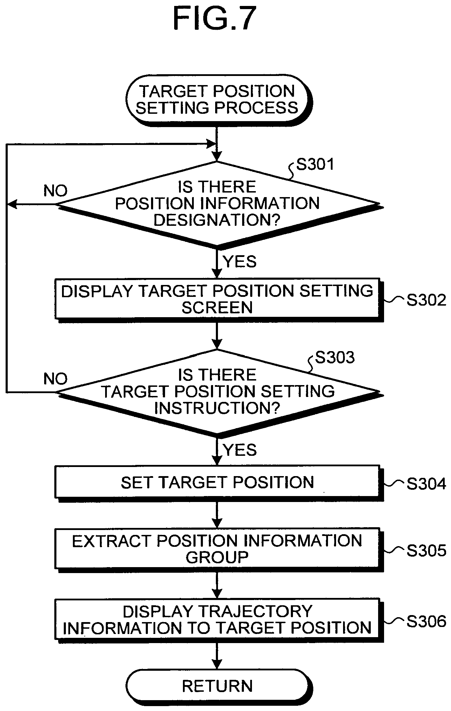

In the following, the target position setting process in step S201 will be described. FIG. 7 is a flowchart exemplifying the operation procedure of the control unit of the capsule guiding system according to the second embodiment until achieving the target position setting process.

In the capsule guiding system 31 during performing the target position setting process of step S201, the control unit 39 firstly determines whether or not there is designation of the position information on the movement trajectory of the capsule medical device 2 (step S301) as indicated in FIG. 7. In step S301, the input unit 7 inputs, to the control unit 39, the position designation information designating a desired positional point which is selected by input operation of the user from among each of position information in the trajectory information of the capsule medical device 2 displayed on the display unit 8, that is, respective positional points forming the movement trajectory of the capsule medical device 2. When the position designation information is acquired from the input unit 7, the control unit 39 determines that there is designation of the position information corresponding to the acquired position designation information ("Yes" in step S301). Then, the control unit 39 controls the display unit 8 to display a setting screen of the target position of the capsule medical device 2 for the backward guiding (step S302).

In step S302, the control unit 39 causes the display unit 8 to display the setting screen inquiring with the user whether or not the position information designated by the position designation information in step S301 is set to be the target position of the capsule medical device 2 for the backward guiding. In this case, the display unit 8 displays the setting screen on which setting or non-setting of the target position can be selected for the user with the operation of the input unit 7, such that the setting screen includes a selection icon of "Setting (Yes)" and a selection icon of "Non-setting (No)", for example.

Subsequently, the control unit 39 determines whether or not there is a setting instruction of the target position of the capsule medical device 2 for the backward guiding (step S303). In step S303, when setting instruction information of the target position of the capsule medical device 2 for the backward guiding is input by the input unit 7, the control unit 39 determines that there is a setting instruction of the target position. When non-setting instruction information of the target position is input by the input unit 7, the control unit 39 determines that there is no setting instruction of the target position. Here, the input unit 7 inputs the setting instruction information of the target position to the control unit 39 in response to the operation of clicking the selection icon of "Setting (Yes)" in the setting screen displayed on the display unit 8, for example. On the contrary, the input unit 7 inputs the non-setting instruction information of the target position to the control unit 39 in response to the operation of clicking the selection icon of "Non-setting (No)" in the setting screen displayed on the display unit 8, for example.

When it is determined that there is the setting instruction of the target position in step S303 ("Yes" in step S303), the control unit 39 sets the target position of the capsule medical device 2 for the backward guiding (step S304). In step S304, the control unit 39 extracts the position information designated by the position designation information in step S301 from among the position information group 9b in the storage unit 9 and sets the extracted position information as the target position of the capsule medical device 2 for the backward guiding.

Next, the control unit 39 extracts the position information group corresponding to the movement trajectory from the target position set in step S304 to the current position of the capsule medical device 2 from among the position information group 9b in the storage unit 9 (step S305). Subsequently, the control unit 39 controls the display unit 8 to display the trajectory information from the current position to the target position of the capsule medical device 2 based on the position information group extracted in step S305 (step S306), and then, returns to step S201 in FIG. 6.

In step S306, the control unit 39 highlights the trajectory information formed by the position information group extracted in step S305, that is, the trajectory information of a section from the current position to the target position of the capsule medical device 2 with flashing or display color changing etc. among the trajectory information of the capsule medical device 2 displayed on the display unit 8.

When the position designation information is not acquired from the input unit 7 in step S301, the control unit 39 determines that there is no designation of the position information ("No" in step S301) and repeats step S301. Further, when it is determined that there is no setting instruction of the target position in step S303 ("No" in step S303), the control unit 39 deletes the currently-acquired position designation information, and then, the operation procedure of step S301 and thereafter is repeated as returning to step S301.

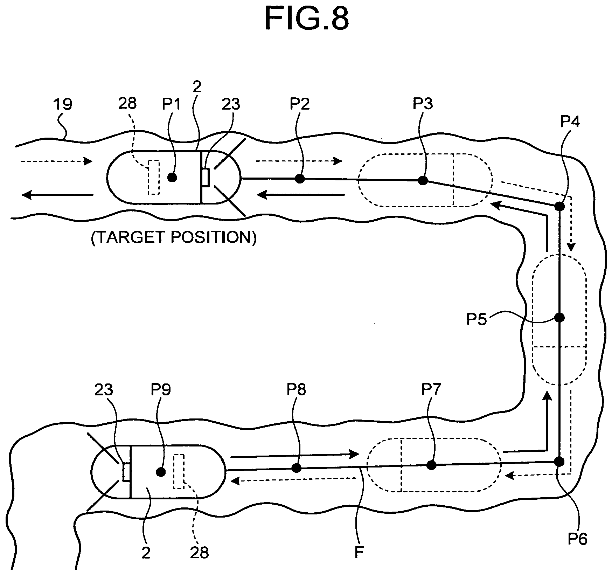

In the following, the operation of the capsule guiding system 31 according to the second embodiment will be specifically described with an example in which the capsule medical device 2 located at the inside of a small intestine of the subject is backwardly guided to the target position. FIG. 8 is a schematic view exemplifying a state that the capsule medical device in the small intestine backwardly moves to the target position on the own movement trajectory.

As indicated by dotted line arrows in FIG. 8, the operation of the capsule guiding system 31 until magnetically guiding the capsule medical device 2 to the current position P9 sequentially via the positions P1 to P8 is similar to that of the capsule guiding system 1 according to the first embodiment. Therefore, in the following, the operation of the capsule guiding system 31 to set the target position for backwardly guiding the capsule medical device 2 and to backwardly guide the capsule medical device 2 to the set target position will be described with reference to FIGS. 5 and 8.

As illustrated in FIG. 8, the control unit 39 of the capsule guiding system 31 sets the past position P1 on the movement trajectory F of the capsule medical device 2 as the target position for the backward guiding by performing the target position setting process. Then, in the case that the backward moving instruction information is input by the input unit 7, the control unit 39 controls the magnetic guiding device 4 so that the capsule medical device 2 backwardly moves on the movement trajectory F from the current position P9 to the past position P1 which is the target position.

Specifically, the control unit 39 firstly extracts the guidance information associated with the position information of the position P9 and the guidance information associated with the position information of the position P8 from among the guidance information group 9a in the storage unit 9. The magnetic field information calculator 10c calculates the magnetic field intensity and the magnetic field direction of the magnetic field for guidance in order to backwardly guide the capsule medical device 2 from the position P9 to the position P8 based on each of the extracted guidance information. The control unit 39 controls the magnetic guiding device 4 so that the magnetic field for guidance having the calculated magnetic field intensity and magnetic field direction is to be generated. The magnetic guiding device 4 applies the magnetic field for guidance for the backward guiding to the capsule medical device 2 based on the control of the control unit 39, thereby backwardly guiding the capsule medical device 2 from the position P9 to the position P8.

In a section from the position P9 to the position P8, the distance calculator 39d calculates the deviation distance between the current position and the movement trajectory F of the capsule medical device 2 based on the position information at the current time of the capsule medical device 2 in a state of being backwardly guided and each of position information of the positions P8 and P9. The control unit 39 controls the magnetic guiding device 4 so that the backward guiding of the capsule medical device 2 is continued when the calculated deviation distance is equal to or smaller than the threshold value and the backward guiding of the capsule medical device 2 is stopped when the calculated deviation distance exceeds the threshold value.

Further, the distance calculator 39d calculates the remaining distance from the current position to the target position P1 of the capsule medical device 2 based on the position information at the current time of the capsule medical device 2 in a state of being backwardly guided and each of position information of the positions P1 to P9. The control unit 39 controls the magnetic guiding device 4 so that the backward guiding of the capsule medical device 2 is continued when the calculated remaining distance exceeds the threshold value and the backward guiding of the capsule medical device 2 is stopped when the calculated remaining distance is equal to or smaller than the threshold value. Here, in the section from the position P9 to the position P8, the control unit 39 continues the backward guiding of the capsule medical device 2 as the remaining distance exceeds the threshold value.

Subsequently, the control unit 39 extracts the guidance information associated with the position information of the position P8 and the guidance information associated with the position information of the position P7 from among the guidance information group 9a in the storage unit 9. The magnetic field information calculator 10c calculates the magnetic field intensity and the magnetic field direction of the magnetic field for guidance in order to backwardly guide the capsule medical device 2 from the position P8 to the position P7 based on each of the extracted guidance information. The control unit 39 controls the magnetic guiding device 4 so that the magnetic field for guidance having the calculated magnetic field intensity and magnetic field direction is to be generated. The magnetic guiding device 4 applies the magnetic field for guidance for the backward guiding to the capsule medical device 2 based on the control of the control unit 39, thereby backwardly guiding the capsule medical device 2 from the position P8 to the position P7.

In a section from the position P8 to the position P7, the distance calculator 39d calculates the deviation distance between the current position and the movement trajectory F of the capsule medical device 2 based on the position information at the current time of the capsule medical device 2 in a state of being backwardly guided and each of position information of the positions P7 and P8. The control unit 39 controls the magnetic guiding device 4 so that the backward guiding of the capsule medical device 2 is continued when the calculated deviation distance is equal to or smaller than the threshold value and the backward guiding of the capsule medical device 2 is stopped when the calculated deviation distance exceeds the threshold value.

Further, the distance calculator 39d calculates the remaining distance from the current position to the target position P1 of the capsule medical device 2 based on the position information at the current time of the capsule medical device 2 in a state of being backwardly guided and each of position information of the positions P1 to P9. The control unit 39 controls the magnetic guiding device 4 so that the backward guiding of the capsule medical device 2 is continued when the calculated remaining distance exceeds the threshold value and the backward guiding of the capsule medical device 2 is stopped when the calculated remaining distance is equal to or smaller than the threshold value. Here, in the section from the position P8 to the position P7, the control unit 39 continues the backward guiding of the capsule medical device 2 as the remaining distance exceeds the threshold value.

Subsequently, the control unit 39 repeats the processes similarly to the case to backwardly guide the capsule medical device 2 from the position P9 to the position P7 via the position P8 also for each section from the position P7 to the target position P1 to cause the magnetic guiding device 4 to sequentially generate the magnetic field for guidance required for the backward guiding of the capsule medical device 2. The magnetic guiding device 4 backwardly guides the capsule medical device 2 so that the capsule medical device 2 backwardly moves on the movement trajectory F toward the target position P1 based on the control of the control unit 39.

Here, the remaining distance to the target position P1 calculated by the distance calculator 39d becomes equal to or smaller than the threshold value when the capsule medical device 2 arrives at the position P1. In this case, the control unit 39 determines that the capsule medical device 2 has arrived at the target position P1 based on the fact that the remaining distance is equal to or smaller than the threshold value. The control unit 39 causes the display unit 8 to display the arrival information and controls the magnetic guiding device 4 to stop the backward guiding of the capsule medical device 2.

As indicated by solid line arrows in FIG. 8, the capsule medical device 2 backwardly guided as described above backwardly moves on the movement trajectory F from the position P9 which is the starting point of the backward guiding and arrives at the target position P1 as sequentially passing through the positions P8, P7, P6, P5, P4, P3, and P2 in FIG. 8 in this order. In this case, the capsule medical device 2 performs backward moving operation in a state that the imaging field of the imaging unit 23 is directed to a direction being different from the backward direction, that is, to the opposite direction.

When the deviation distance calculated by the distance calculator 39d exceeds the threshold value at any section from the position P9 to the position P1 in FIG. 8, the control unit 39 determines that the position deviation between the current position of the capsule medical device 2 in the state of being backwardly guided and the movement trajectory F exceeds an assumed range. In this case, even before the capsule medical device 2 arrives at the target position P1, the control unit 39 causes the display unit 8 to display the stop information and controls the magnetic guiding device 4 to stop the backward guiding of the capsule medical device 2.

As described above, in the second embodiment of the present invention, position information of a capsule medical device in a subject is sequentially detected by a position detection device in chronological order. Then, a storage unit sequentially stores in chronological order each of guidance information of a magnetic guiding device during the capsule medical device is magnetically guided in association with each of position information of the capsule medical device detected by the position detection device. A target position for backward guiding is selectively set among a position information group of the capsule medical device detected by the position detection device and the capsule medical device is to be backwardly guided to the set target position. The rest thereof is structured similarly to the first embodiment. Accordingly, the functions and effects being similar to those of the above first embodiment are obtained. In addition, it is possible to actualize a capsule guiding system in which a capsule medical device can be backwardly moved to a desired target position in a subject without increasing operational burden for a user, and in which easy selecting of a desired position from among respective positions on a movement trajectory of the capsule medical device and setting of the selected desired position as the target position for backward guiding are enabled.

### Third Embodiment

In the following, a third embodiment of the present invention will be described. In the above second embodiment, a desired position selected from among trajectory information of the capsule medical device 2 displayed on the display unit 8 is set as the target position for backward guiding. In the third embodiment, in-vivo images taken by the capsule medical device 2, guidance information of the magnetic guiding device 4 and position information detected by the position detection device 5 are sequentially stored in chronological order and in association with each other, and the capsule medical device 2 is backwardly guided to a target position based on the stored in-vivo image groups and the associated guidance information group.

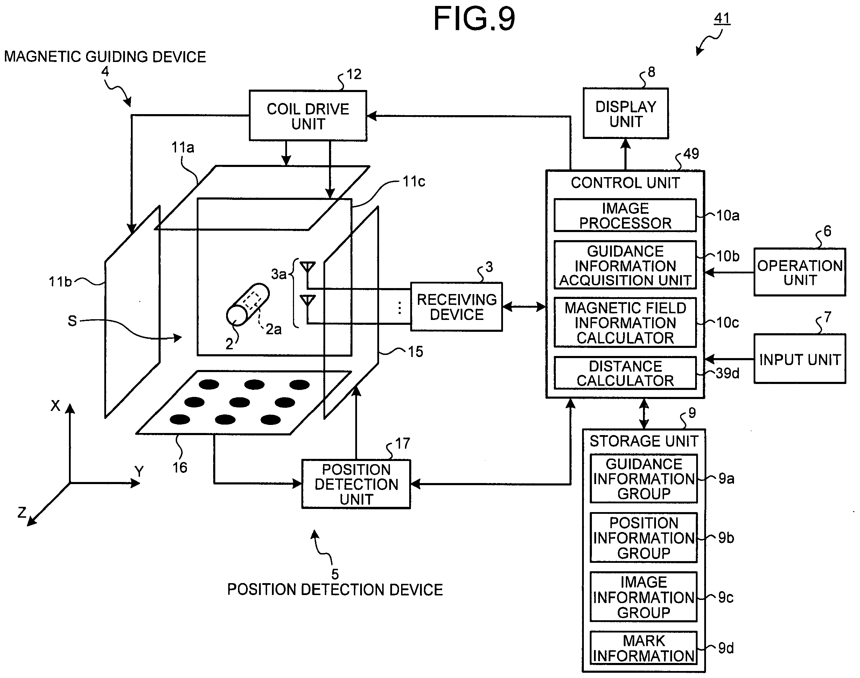

FIG. 9 is a schematic view schematically illustrating a structural example of a capsule guiding system according to the third embodiment of the present invention. As illustrated in FIG. 9, a capsule guiding system 41 according to the third embodiment is provided with a control unit 49 instead of the control unit 39 of the capsule guiding system 31 according to the above second embodiment. Further, in the capsule guiding system 41, the input unit 7 inputs mark information 9d to be assigned to an in-vivo image displayed on the display unit 8 and the storage unit 9 stores the mark information 9d in a manner of being associated with the in-vivo image. The rest of the structure is the same as the second embodiment and the same reference numeral is given to the same structural part.

The control unit 49 controls the storage unit 9 to store each of guidance information of the magnetic guiding device 4 sequentially acquired by the guidance information acquisition unit 10b, each of position information of the capsule medical device 2 sequentially detected by the position detection unit 17, and image information of each in-vivo image sequentially taken by the capsule medical device 2 in chronological order and in association with each other. That is, each of guidance information included in the guidance information group 9a, each of position information included in the position information group 9b and each in-vivo image included in the image information group 9c in the storage unit 9 are respectively associated in chronological order by the control unit 49.

Further, when the mark information 9d is input by the input unit 7, the control unit 49 assigns the mark information 9d to the in-vivo image currently displayed on the display unit 8. The control unit 49 controls the storage unit 9 to store the mark information 9d in association with the image information of the in-vivo image. The control unit 49 extracts, from the storage unit 9, the guidance information of the magnetic guiding device 4 that is guidance information for magnetically guiding the capsule medical device 2 from a past position on the movement trajectory, which is the imaging position of the in-vivo image and to which the mark information 9d is assigned, to the current position. Then, the control unit 49 controls the magnetic guiding device 4 to backwardly guide the capsule medical device 2 from the current position to the past position based on the extracted guidance information. In this case, the control unit 49 sets the position information which corresponds to the imaging position of the in-vivo image to which the mark information is assigned as the target position for the backward guiding. Here, other functions of the control unit 49 are similar to those of the control unit 39 in the capsule guiding system 31 according to the above second embodiment.

In the third embodiment, the input unit 7 inputs the mark information 9d to be assigned to the in-vivo image to the control unit 49 in response to predetermined input operation, such as clicking while locating a cursor at the in-vivo image currently displayed on the display unit 8.

When the capsule medical device 2 in the subject is backwardly guided, the control unit 49 appropriately repeats the operation procedure which is approximately similar to steps S201 to S212 in FIG. 6. In step S201, the control unit 49 performs a target position setting process which is different from that by the control unit 39 of the second embodiment, that is, performs the target position setting process to set the imaging position of the in-vivo image to which the mark information 9d is assigned as the target position for the backward guiding. Meanwhile, in step S203, the control unit 49 sequentially extracts, in reverse chronological order, the position information to form the movement trajectory from the imaging position of the in-vivo image to which the mark information 9d is assigned (i.e., the target position for the backward guiding) to the current position from among the position information group 9b in the storage unit 9. Further, the control unit 49 sequentially extracts, in reverse chronological order, the guidance information associated with the position information extracted from the position information group 9b from among the guidance information group 9a in the storage unit 9. That is, the control unit 49 sequentially extracts, in reverse chronological order, each of guidance information from the guidance information associated with the in-vivo image to which the mark information 9d is assigned through the current guidance information from among the guidance information group 9a.

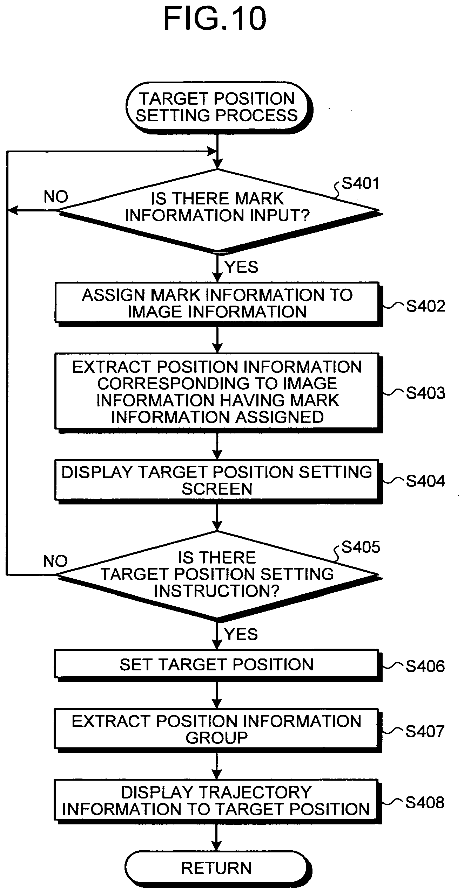

Next, the target position setting process of the third embodiment will be described. FIG. 10 is a flowchart exemplifying operation procedure until the control unit of the capsule guiding system according to the third embodiment achieves the target position setting process.

When the capsule guiding system 41 performs the target position setting process in the operation procedure of step S201 in FIG. 6, the control unit 49 firstly determines whether or not there is input of the mark information 9d to be assigned to the in-vivo image displayed on the display unit 8 (step S401) as indicated in FIG. 10. In step S401, when the mark information 9d is input by the input unit 7, the control unit 49 determines that there is input of the mark information 9d ("Yes" in step S401) and assigns the acquired mark information 9d to the image information of the in-vivo image (step S402).

In step S402, the control unit 49 assigns the mark information 9d to the image information of the in-vivo image currently displayed on the display unit 8 and controls the display unit 8 to display the mark information 9d as being superimposed with the in-vivo image while controlling the storage unit 9 to store the mark information 9d in association with the image information of the current in-vivo image.

Subsequently, the control unit 49 extracts the position information corresponding to the image information of the in-vivo image to which the mark information 9d is assigned in step S402 (step S403). In this case, the control unit 49 extracts the position information associated with the image information of the in-vivo image to which the mark information 9d is assigned from among the position information group 9b in the storage unit 9.

Then, the control unit 49 controls the display unit 8 to display a setting screen of the target position of the capsule medical device 2 for the backward guiding (step S404). In step S404, the control unit 49 causes the display unit 8 to display the setting screen inquiring with the user whether or not the imaging position of the in-vivo image to which the mark information 9d is assigned in step S401 is set to be the target position of the capsule medical device 2 for the backward guiding. In this case, similarly to the case of the above second embodiment, the display unit 8 displays the setting screen on which setting or non-setting of the target position can be selected for the user with the operation of the input unit 7.

Subsequently, similarly to step S303 in FIG. 7, the control unit 49 determines whether or not there is a setting instruction of the target position of the capsule medical device 2 for the backward guiding (step S405). When it is determined that there is the setting instruction of the target position ("Yes" in step S405), the control unit 49 sets the target position of the capsule medical device 2 for the backward guiding (step S406). In step S406, the control unit 49 sets the imaging position of the in-vivo image to which the mark information 9d is assigned in step S401, that is, the position information extracted in step S403, as the target position of the capsule medical device 2 for the backward guiding.

Next, the control unit 49 extracts the position information group corresponding to the movement trajectory from the target position set in step S406 to the current position of the capsule medical device 2 from among the position information group 9b in the storage unit 9 (step S407). Subsequently, the control unit 49 controls the display unit 8 to display the trajectory information from the current position to the target position of the capsule medical device 2 based on the position information group extracted in step S407 (step S408), and then, returns to step S201 in FIG. 6.

In step S408, the control unit 49 highlights the trajectory information formed by the position information group extracted in step S407, that is, the trajectory information of a section from the current position to the target position of the capsule medical device 2 with flashing or display color changing etc. from among the trajectory information of the capsule medical device 2 displayed on the display unit 8.

Incidentally, when the mark information 9d is not acquired from the input unit 7 in step S401, the control unit 49 determines that there is no input of the mark information 9d ("No" in step S401) and repeats step S401. Further, when it is determined that there is no setting instruction of the target position in step S405 ("No" in step S405), the control unit 49 deletes the currently-acquired position designation information, and then, the operation procedure of step S401 and thereafter is repeated as returning to step S401.

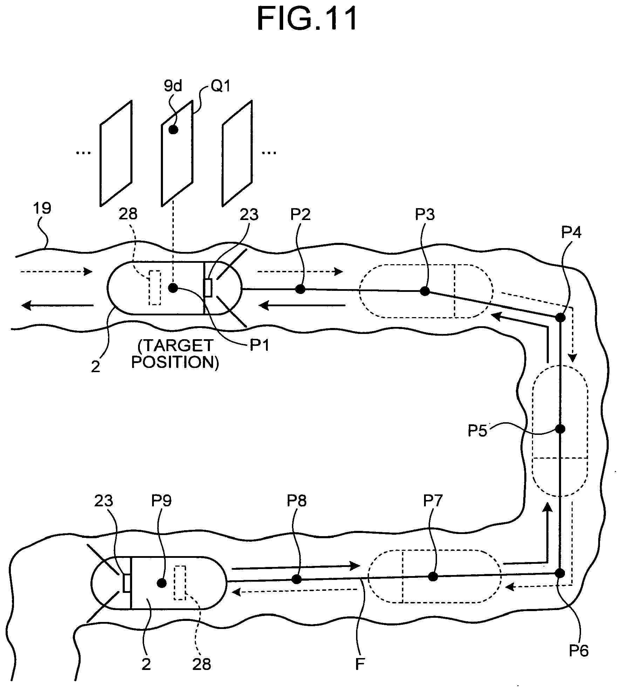

In the following, the operation of the capsule guiding system 41 according to the third embodiment will be specifically described with an example in which the capsule medical device 2 located at the inside of a small intestine of the subject is backwardly guided to the target position. FIG. 11 is a schematic view exemplifying a state that the capsule medical device backwardly moves on the own movement trajectory to the target position which is the imaging position of the in-vivo image to which the mark information is assigned. Here, the operation of the capsule guiding system 41 according to the third embodiment is similar to that of the capsule guiding system 31 according to the second embodiment except for the operation to set the imaging position of the in-vivo image to which the mark information 9d is assigned as the target position. Accordingly, in the following, the operation of the capsule guiding system 41 to set the imaging position of the in-vivo image Q1 to which the mark information 9d is assigned as the target position during the backward guiding will be described with reference to FIGS. 9 and 11.

In the capsule guiding system 41 to perform the target position setting process, the control unit 49 assigns the mark information 9d to the in-vivo image Q1 which is currently displayed on the display unit 8 when the mark information 9d is input by the input unit 7. Then, the control unit 49 sets the imaging position of the in-vivo image Q1 to which the mark information 9d is assigned as the target position for the backward guiding. Specifically, the control unit 49 extracts the position information which is associated with the image information of the in-vivo image Q1 to which the mark information 9d is assigned, that is, the position information of the imaging position of the in-vivo image Q1, from among the position information group 9b in the storage unit 9. Then, the control unit 49 sets the position P1 on the movement trajectory F corresponding to the extracted position information as the target position.

Subsequently, in the case that the backward moving instruction information is input by the input unit 7, the control unit 49 controls the magnetic guiding device 4 so that the capsule medical device 2 backwardly moves on the movement trajectory F from the current position P9 to the past position P1 which is the target position as repeating the processes similar to those in the above second embodiment. The magnetic guiding device 4 applies the magnetic field for guidance to the capsule medical device 2 at the inside of the small intestine 19 based on the control of the control unit 49, thereby backwardly guiding the capsule medical device 2 to the position P1 which is the target position.

As indicated by solid line arrows in FIG. 11, the capsule medical device 2 backwardly guided as described above backwardly moves on the movement trajectory F from the position P9 which is the starting point of the backward guiding and arrives at the target position P1 as sequentially passing through the positions P8, P7, P6, P5, P4, P3, and P2 in FIG. 11 in this order.

Here, the position P1 which is the target position is approximately matched with the imaging position of the in-vivo image Q1 to which the mark information 9d is assigned. The capsule medical device 2, which has arrived at the position P1, takes an in-vivo image that is similar to the in-vivo image Q1 with the imaging unit 23 in a state that the imaging field of the imaging unit 23 is directed to the direction almost the same as that when the in-vivo image Q1 was taken. The in-vivo image taken as described above by the capsule medical device 2 at the position P1 is displayed on the display unit 8. The user can easily observe once again the vicinity of the imaging position of the in-vivo image Q1, to which the mark information 9d is assigned, by observing the in-vivo image displayed on the display unit 8 as described above.

In the third embodiment of the present invention, as described above, the image information of each in-vivo image in the subject taken by the capsule medical device, each of guidance information of the magnetic guiding device during the magnetic guiding of the capsule medical device, and each of the position information of the capsule medical device detected by the position detection device are sequentially stored in the storage unit in chronological order and in association with each other. The mark information to be assigned to the in-vivo image which is currently displayed on the display unit is input by the input unit. Then, each of guidance information from the guidance information associated with the image information of the in-vivo image to which the mark information is assigned until the current guidance information is sequentially extracted in reverse chronological order. The capsule medical device is to be backwardly guided to the imaging position, that is, the target position, based on the extracted guidance information. The rest thereof is structured similarly to the second embodiment. Accordingly, the functions and effects being similar to those of the above second embodiment are obtained. In addition, it is possible to actualize a capsule guiding system in which a capsule medical device can be backwardly moved with ease to an imaging position of a desired in-vivo image (e.g., a characteristic in-vivo image capturing a bleeding site or an affected site etc.) selected from among an in-vivo image group displayed on a display unit without increasing operational burden for the user.

Further, since the position information associated with the image information of the in-vivo image to which the mark information is assigned is set as the position information of the target position for the backward guiding, the trajectory information of the capsule medical device including the position information of the imaging position can be easily displayed on the display unit while the imaging position of the in-vivo image to which the mark information is assigned can be easily set as the target position for the backward guiding. Accordingly, the user can easily recognize to which position the target position as being the imaging position of the in-vivo image corresponds on the movement trajectory of the capsule medical device.

### Fourth Embodiment

In the following, a fourth embodiment of the present invention will be described. In the above third embodiment, backward guiding speed of the capsule medical device 2 is not particularly controlled. In the fourth embodiment, the backward moving speed of the capsule medical device 2 is changed to low speed when the capsule medical device 2 backwardly moves to the vicinity of the target position.

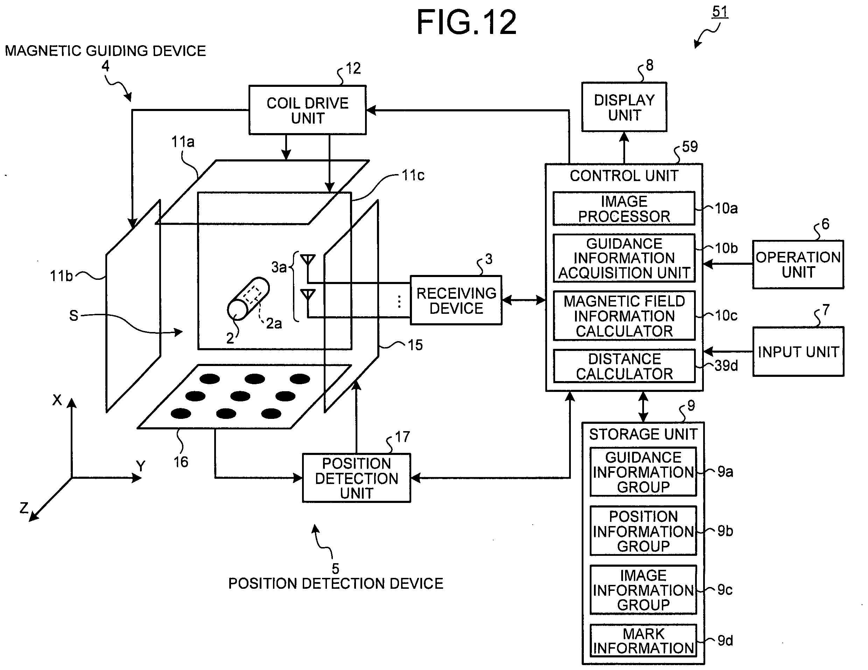

FIG. 12 is a schematic view schematically illustrating a structural example of a capsule guiding system according to the fourth embodiment of the present invention. As illustrated in FIG. 12, a capsule guiding system 51 according to the fourth embodiment is provided with a control unit 59 instead of the control unit 49 of the capsule guiding system 41 according to the above third embodiment. The rest of the structure is the same as the third embodiment and the same reference numeral is given to the same structural part.

The control unit 59 has a function to control movement speed of the capsule medical device 2 during the backward guiding, that is, magnetic guiding speed to backwardly guide the capsule medical device 2 by the magnetic guiding device 4 (hereinafter, called backward guiding speed). Specifically, as described above, the distance calculator 39d appropriately calculates the remaining distance from the current position to the target position of the capsule medical device 2 during the backward guiding and the deviation distance between the current position of the capsule medical device 2 and the own movement trajectory during the backward guiding. The control unit 59 compares the remaining distance calculated by the distance calculator 39d with a distance set value which is previously set. When the remaining distance exceeds the distance set value, the control unit 59 controls the backward guiding speed of the magnetic guiding device 4 at predetermined normal speed. On the contrary, when the remaining distance is equal to or smaller than the distance set value, the control unit 59 controls the backward guiding speed of the magnetic guiding device 4 to low speed compared to the normal speed. Here, in addition to the function of controlling the backward guiding speed, the control unit 59 has similar functions to those of the control unit 49 in the capsule guiding system 41 according to the above third embodiment.

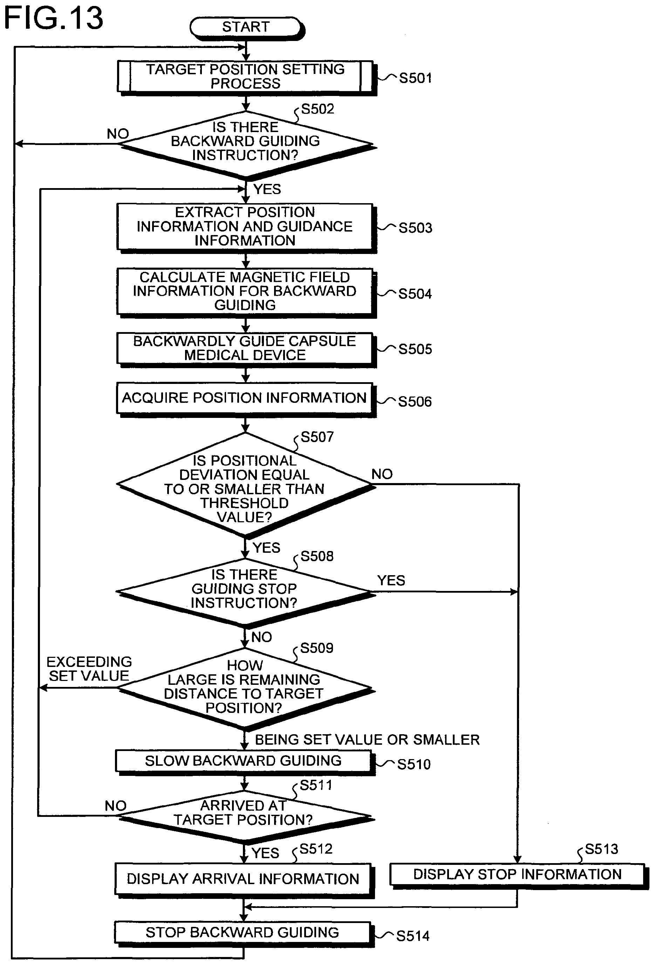

Next, the operation of the capsule guiding system 51 to backwardly guide the capsule medical device 2 in the subject will be described. FIG. 13 is a flowchart exemplifying operation procedure of the capsule guiding system to backwardly guide the capsule medical device in the subject to the target position while controlling the backward guiding speed.

In the capsule guiding system 51 when the capsule medical device 2 in the subject is backwardly guided, the control unit 59 firstly performs a target position setting process to set the target position of the capsule medical device 2 for the backward guiding (step S501), as indicated in FIG. 13. Then, similarly to step S202 as indicated in FIG. 6, it is determined whether or not there is an instruction of the backward guiding (step S502). In step S501, the control unit 59 appropriately repeats the operation procedure of steps S401 to S408 in FIG. 10 similarly to the control unit 49 in the above third embodiment.

When it is determined that there is no backward guiding instruction in step S502 ("No" in step S502), as returning to step S501, the control unit 59 repeats operation procedure of step S501 and step S502 until the backward moving instruction information is input by the input unit 7. In this state, the control unit 59 is capable of controlling magnetic guiding of the capsule medical device 2 in the subject with the magnetic guiding device 4 in response to manual operation of the operation unit 6 by the user.

On the contrary, when it is determined that there is a backward guiding instruction ("Yes" in step S502), the control unit 59 extracts the position information of the movement trajectory of the capsule medical device 2 in the subject and the guidance information of the magnetic guiding device 4 from the storage unit 9 (step S503) similarly to the control unit 49 of the above third embodiment.

Next, similarly to step S204 and step S205 in FIG. 6, the control unit 59 calculates the magnetic field information of the magnetic field for guidance required for backwardly guiding the capsule medical device 2 based on the guidance information extracted in step S503 (step S504). Then, the control unit 59 controls the backward guiding of the capsule medical device 2 in the subject based on the magnetic field information calculated in step S504, that is, the magnetic field intensity information and the magnetic field direction information of the magnetic field for guidance (step S505). Subsequently, similarly to step S206 in FIG. 6, the control unit 59 acquires the position information of the capsule medical device 2 backwardly guided in step S505 (step S506).

Then, similarly to step S207 in FIG. 6, the control unit 59 determines whether or not positional deviation between the current position of the backwardly-guided capsule medical device 2 and the own movement trajectory is equal to or smaller than the predetermined threshold value (step S507). When it is determined that the positional deviation is equal to or smaller than the threshold value ("Yes" in step S507), the control unit 59 determines whether or not there is an instruction to stop guiding (step S508) similarly to step S208 in FIG. 6. When it is determined that there is no instruction to stop guiding in step S508 ("No" in step S508), the control unit 59 evaluates the remaining distance from the current position of the capsule medical device 2 to the target position (step S509).

In step S509, the distance calculator 39d calculates the remaining distance from the current position to the target position of the capsule medical device 2 for the backward guiding based on the current position information acquired from the position detection unit 17 in step S506 and each of position information to the target position set in step S501. The control unit 59 performs a comparison process between the remaining distance calculated by the distance calculator 39d and the distance set value which is previously set. When the remaining distance exceeds the distance set value (i.e., exceeding the set value in step S509), the control unit 59 determines that the capsule medical device 2 has not arrived yet at the vicinity of the target position, and then, the control unit 59 repeats the operation procedure of step S503 and thereafter as returning to step S503. In this case, the control unit 59 maintains the backward guiding speed of the magnetic guiding device 4 to backwardly guide the capsule medical device 2 at the normal speed.

Here, the normal speed denotes the normal backward guiding speed during backwardly guiding the capsule medical device 2 in the section from the starting position to the vicinity of the target position (i.e., the position of which remaining distance to the target position is equal to or smaller than the set distance) of the backward guiding.

On the contrary, when the remaining distance is equal to or smaller than the distance set value in step S509 (i.e., being the set value or smaller in step S509), the control unit 59 determines that the capsule medical device 2 has arrived at the vicinity of the target position and slows the backward guiding of the capsule medical device 2 (step S510). In step S510, the control unit 59 controls the backward guiding speed of the magnetic guiding device 4 at low speed compared to the normal speed by slowing reading process speed of the position information or guidance information or by reducing the magnetic field intensity of the magnetic field for guidance by a fixed rate. Accordingly, the movement speed of the capsule medical device 2 in backward guiding is slowed.

Subsequently, similarly to step S209 in FIG. 6, the control unit 59 determines whether or not the capsule medical device 2 has arrived at the target position (step S511). When it is determined that the capsule medical device 2 has not arrived yet at the target position ("No" in step S511), the control unit 59 repeats the operation procedure of step S503 and thereafter as returning to step S503. On the contrary, when it is determined that the capsule medical device 2 has arrived at the target position ("Yes" in step S511), the control unit 59 controls the display unit 8 to display the arrival information indicating that the capsule medical device 2 has arrived at the target position (step S512) similarly to step S210 in FIG. 6.

Then, the control unit 59 controls the magnetic guiding device 4 to stop the backward guiding of the capsule medical device 2 (step S514) similarly to step S212 in FIG. 6. Then, the control unit 59 repeats the operation process of step S501 and thereafter as returning to step S501. At that time, the capsule medical device 2 in the subject is in a state of having been backwardly moved to the target position which is set with the target position setting process in step S501. Meanwhile, the user recognizes that the capsule medical device 2 has backwardly moved to the desired target position by observing the arrival information displayed on the display unit 8.

On the contrary, when it is determined that the positional deviation between the current position of the capsule medical device 2 and the own movement trajectory exceeds the threshold value in step S507 ("No" in step S507) or it is determined that there is an instruction to stop guiding in step S508 ("Yes" in step S508), the control unit 59 controls the display unit 8 to display stop information indicating to stop the backward guiding of the capsule medical device 2 (step S513) similarly to step S211 in FIG. 6. Then, the control unit 59 repeats the operation procedure of step S514 and thereafter as proceeding to step S514. At that time, the user recognizes that the backward moving operation is stopped without arriving of the capsule medical device 2 at the desired target position by observing the stop information displayed at the display unit 8.

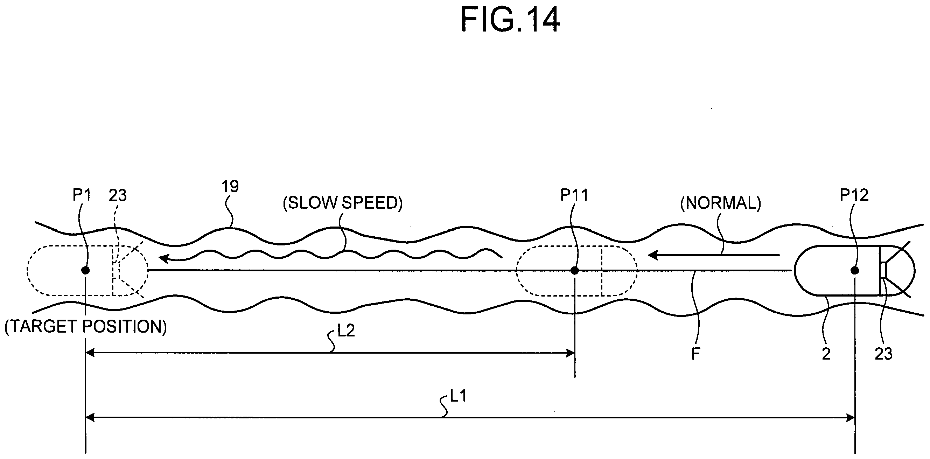

In the following, the operation of the capsule guiding system 51 according to the fourth embodiment will be specifically described with an example in which the capsule medical device 2 located at the inside of a small intestine of the subject is backwardly guided to the target position. FIG. 14 is a schematic view exemplifying a state that the capsule medical device is backwardly guided as slowing the backward guiding speed in the vicinity of the target position. Here, the operation of the capsule guiding system 51 according to the fourth embodiment is similar to that of the capsule guiding system 41 according to the third embodiment except for the operation to slow the backward guiding speed of the magnetic guiding device 4 which backwardly guides the capsule medical device 2. Accordingly, in the following, the operation of the capsule guiding system 51 to backwardly guide the capsule medical device 2 as slowing the backward guiding speed in the vicinity of the target position will be described with reference to FIGS. 12 and 14.

In the capsule guiding system 51 to backwardly guide the capsule medical device 2 in the subject, the control unit 59 monitors the calculation value of the distance calculator 39d, that is, the remaining distance from the current position of the capsule medical device 2 to the position P1 which is the target position at the inside of the small intestine 19 while controlling the magnetic guiding device 4 so that the capsule medical device 2 backwardly moves on the movement trajectory F to the position P1.

Here, as illustrated in FIG. 14, when the capsule medical device 2 backwardly moves to a position P12 on the movement trajectory F, the distance calculator 39d calculates remaining distance L1 from the position P12 being the current position of the capsule medical device 2 to the position P1 being the target position. Since the calculated remaining distance L1 exceeds the predetermined distance set value, the control unit 59 determines that the capsule medical device 2 has not arrived yet at the vicinity of the position P1. In this case, the control unit 59 maintains the backward guiding speed of the magnetic guiding device 4 backwardly guiding the capsule medical device 2 toward the position P1 at the normal speed.

Then, when the capsule medical device 2 backwardly moves to a position P11 on the movement trajectory F, the distance calculator 39d calculates remaining distance L2 from the position P11 being the current position of the capsule medical device 2 to the position P1 being the target position. Since the calculated remaining distance L2 is equal to or smaller than the predetermined distance set value, the control unit 59 determines that the capsule medical device 2 has arrived at the vicinity of the position P1. In this case, the control unit 59 controls the backward guiding speed of the magnetic guiding device 4 backwardly guiding the capsule medical device 2 toward the position P1 to be at low speed compared to the normal speed.

Based on the control of the control unit 59, the magnetic guiding device 4 backwardly guides the capsule medical device 2 at low speed in a section from the position P11 to the position P1. In this case, the capsule medical device 2 sequentially takes in-vivo images with the imaging unit 23 while backwardly moving at the low speed toward the target position P1 from the position P11. The capsule medical device 2 which is backwardly moving at the low speed as described above is capable of circumstantially taking in-vivo images at the vicinity area of the target position P1 compared to the case of backwardly moving at the normal speed.

The user can circumstantially observe the vicinity area of the target position P1 as observing the in-vivo image group taken by the capsule medical device 2 in the low speed backward moving state on the display unit 8. As a result, the user can easily locate a more favorable in-vivo position even when there occurs some positional deviation between the target position which is set by the control unit 59 and a target position which is actually desired to be observed.

In the fourth embodiment of the present invention, as described above, it is determined whether or not the capsule medical device has backwardly moved to the vicinity of the target position based on the remaining distance from the current position to the target position of the capsule medical device in the backward guiding. The backward guiding speed of the magnetic guiding device backwardly guiding the capsule medical device is controlled at the normal speed when the capsule medical device has not backwardly moved yet to the vicinity of the target position. The backward guiding speed of the magnetic guiding device backwardly guiding the capsule medical device is controlled to be at the low speed compared to the normal speed when the capsule medical device has backwardly moved to the vicinity of the target position. The rest thereof is structured similarly to the third embodiment. Accordingly, the functions and effects being similar to those of the above third embodiment are obtained. In addition, it is possible to actualize a capsule guiding system in which circumstantial imaging of an in-vivo image group at a vicinity area of a target position can be performed by a capsule medical device in a low speed backward moving state and easier assisting to locate a more favorable in-vivo position can be performed by displaying the circumstantial in-vivo image group on a display unit even when there occurs some positional deviation between a set target position and an in-vivo position which is actually desired to be observed.

### Fifth Embodiment

In the following, a fifth embodiment of the present invention will be described. In the above fourth embodiment, the backward guiding speed of the capsule medical device 2 is changed to low speed when the capsule medical device 2 has backwardly moved to the vicinity of a target position. In the fifth embodiment, the capsule medical device 2 is further kept staying at the target position by applying a magnetic field to the capsule medical device 2 which has arrived at the target position with the backward guiding.

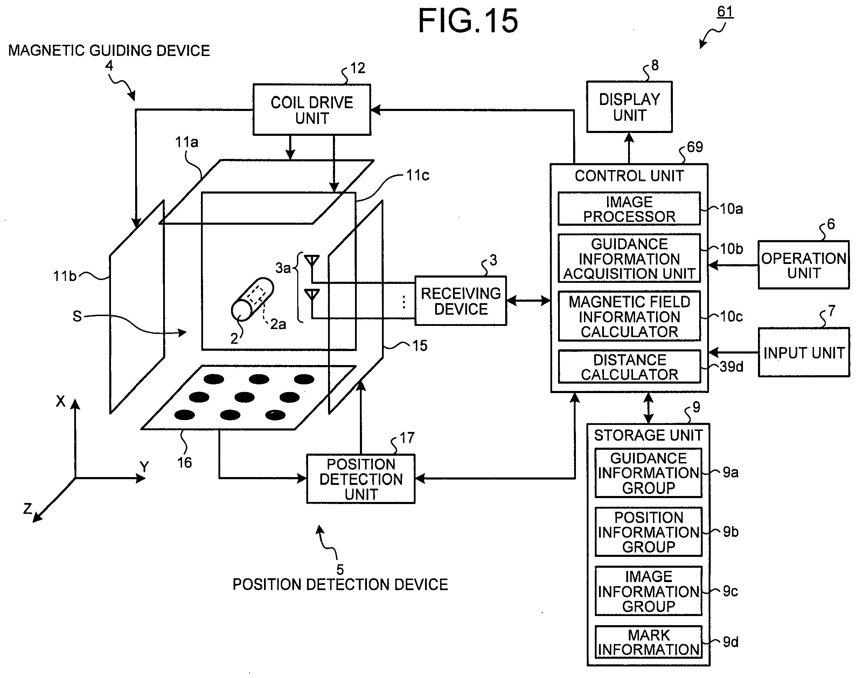

FIG. 15 is a schematic view schematically illustrating a structural example of a capsule guiding system according to the fifth embodiment of the present invention. As illustrated in FIG. 15, a capsule guiding system 61 according to the fifth embodiment is provided with a control unit 69 instead of the control unit 59 in the capsule guiding system 51 according to the above fourth embodiment. The rest of the structure is the same as the fourth embodiment and the same reference numeral is given to the same structural part.

The control unit 69 has a function to control the magnetic guiding device 4 to keep the capsule medical device 2 backwardly guided to the target position in the subject staying at the target position with magnetic force. Specifically, when the capsule medical device 2 arrives at the target position with the backward guiding, the distance calculator 39d sequentially calculates deviation distance (i.e., positional deviation quantity) between the current position and the target position of the capsule medical device 2. The control unit 69 controls the magnetic guiding device 4 to generate the magnetic field for guidance required for keeping the capsule medical device 2 staying at the target position (hereinafter, called the magnetic field for staying) based on the deviation distance calculated by the distance calculator 39d. In this case, the magnetic guiding device 4 magnetically guides the capsule medical device 2 so as to correct the positional deviation between the target position and the current position of the capsule medical device 2 based on the control of the control unit 69, thereby keeping the capsule medical device 2 staying at the target position. Here, in addition to the function of stay control of the capsule medical device 2 at the target position, the control unit 69 has similar functions to those of the control unit 59 in the capsule guiding system 51 according to the above fourth embodiment.

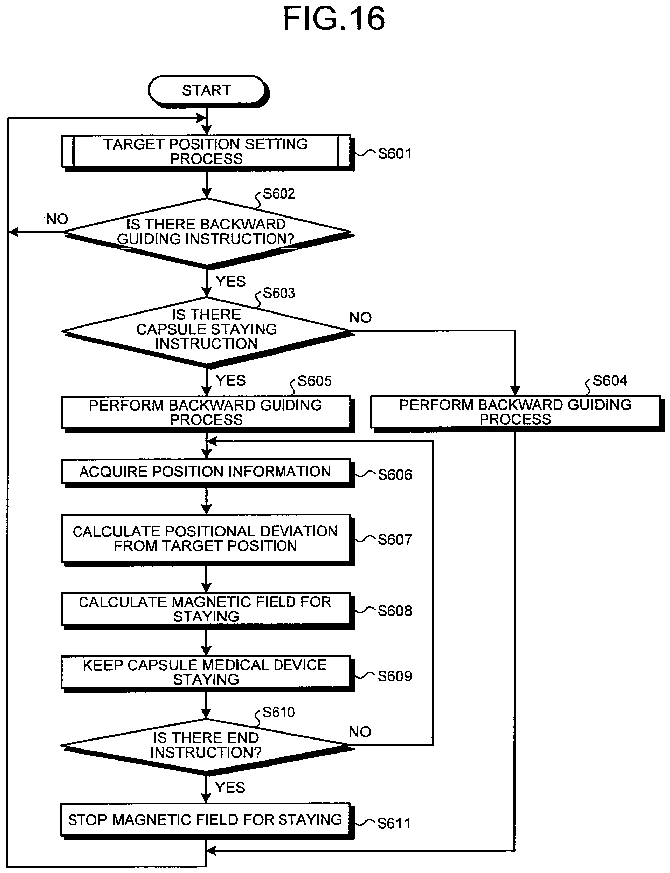

Next, the operation of the capsule guiding system 61 to backwardly guide the capsule medical device 2 in the subject will be described. FIG. 16 is a flowchart exemplifying operation procedure of the capsule guiding system to keep the capsule medical device backwardly guided to the target position in a staying state.

As illustrated in FIG. 16, the control unit 69 in the capsule guiding system 61 firstly performs the target position setting process (step S601) similarly to step S501 in FIG. 13. Then, similarly to step S502 as indicated in FIG. 13, it is determined whether or not there is an instruction of the backward guiding by the control unit 69 (step S602). When it is determined that there is no backward guiding instruction ("No" in step S602), as returning to step S601, the control unit 69 repeats operation procedure of step S601 and step S602 until the backward moving instruction information is input by the input unit 7. In this state, the control unit 69 is capable of controlling magnetic guiding of the capsule medical device 2 in the subject with the magnetic guiding device 4 in response to manual operation of the operation unit 6 by the user.

On the contrary, when it is determined that there is a backward guiding instruction ("Yes" in step S602), the control unit 69 determines whether or not there is an instruction of capsule staying at the target position (step S603). Here, the capsule staying at the target position denotes keeping the capsule medical device 2, which has arrived at the target position set in step S601, staying at the target position. When capsule staying instruction information is not input by the input unit 7, the control unit 69 determines that there is no capsule staying instruction at the target position in step S603.

When it is determined that there is no capsule staying instruction ("No" in step S603), the control unit 69 performs the backward guiding process to backwardly guide the capsule medical device 2 toward the target position (step S604). In step S604, the control unit 69 performs the operation procedure being similar to steps S503 to S514 in FIG. 13. Then, the control unit 69 repeats the operation procedure of step S601 and thereafter as returning to step S601.

On the contrary, when it is determined that there is a capsule staying instruction in step S603 ("Yes" in step S603), the control unit 69 performs the backward guiding process to backwardly guide the capsule medical device 2 toward the target position (step S605), and then acquires, from the position detection unit 17, the position information indicating the current position of the capsule medical device 2 after being backwardly guided (step S606). Here, in step S605, the control unit 69 performs the operation procedure being similar to steps S503 to S514 in FIG. 13.

Then, the control unit 69 calculates the positional deviation of the capsule medical device 2 from the target position in the backward guiding in step S605 (step S607). In step S607, the distance calculator 39d calculates the deviation distance between the target position and the current position of the capsule medical device 2 based on the position information of the target position set in step S601 and the position information acquired in step S606. The control unit 69 acquires the deviation distance calculated by the distance calculator 39d as described above as the position deviation quantity (i.e., positional difference) between the target position and the current position of the capsule medical device 2.

Subsequently, the control unit 69 calculates the magnetic field for staying to keep the capsule medical device 2 staying at the target position (step S608) and controls the magnetic guiding device 4 to keep the capsule medical device 2 staying at the target position by applying the calculated magnetic field for staying to the capsule medical device 2 (step S609).

In step S608 and step S609, the control unit 69 calculates an electric current pattern required for generating the magnetic field for staying based on the positional deviation quantity calculated in step S607 and controls the magnetic guiding device 4 to generate the magnetic field for staying based on the calculated electric current pattern. In this case, the control unit 69 calculates the magnetic field direction of the magnetic field for staying based on each of position information of the target position and the current position of the capsule medical device 2. Further, the control unit 69 utilizes a previously-set defined value as the magnetic field intensity of the magnetic field for staying. The magnetic guiding device 4 applies the magnetic field for staying having the magnetic field direction and the magnetic field intensity to the capsule medical device 2 based on the control of the control unit 69, thereby magnetically guiding the capsule medical device 2 in the direction to correct the positional deviation between the target position and the current position of the capsule medical device 2. As a result, the capsule medical device 2 is in a staying state at the target position even when peristalsis of an alimentary canal occurs, for example.

Subsequently, the control unit 69 determines whether or not there is an end instruction of capsule staying at the target position (step S610). When end instruction information of the capsule staying is input by the input unit 7, the control unit 69 determines that there is an end instruction of capsule staying ("Yes" in step S610) and controls the magnetic guiding device 4 to stop generation of the magnetic field for staying (step S611). As a result, the capsule medical device 2 is released from a state of being constrained by the magnetic field for staying of the magnetic guiding device 4. Then, the control unit 69 repeats the operation procedure of step S601 and thereafter as returning to step S601.

On the contrary, when the end instruction information of the capsule staying is not input in step S610, the control unit 69 determines that there is no end instruction of capsule staying ("No" in step S610). Then, the control unit 69 repeats the operation procedure of step S606 and thereafter as returning to step S606.

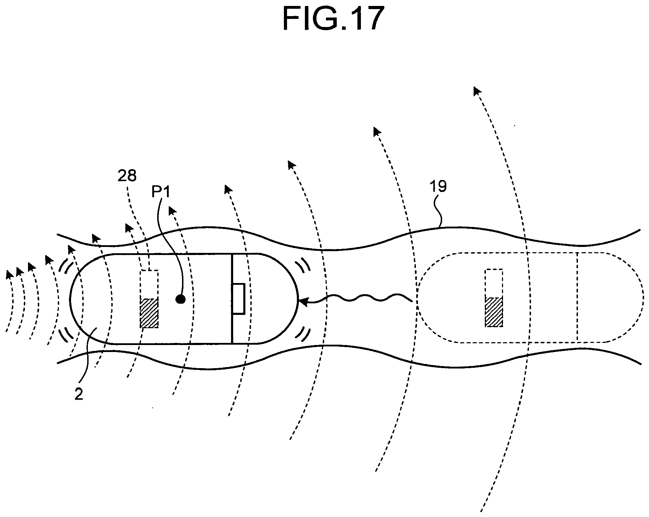

In the following, the operation of the capsule guiding system 61 according to the fifth embodiment will be specifically described with an example in which the capsule medical device 2 located at the inside of a small intestine of the subject is backwardly guided to the target position. FIG. 17 is a schematic view exemplifying a state that the capsule medical device is kept staying at the target position. Here, the operation of the capsule guiding system 61 according to the fifth embodiment is similar to that of the capsule guiding system 51 according to the fourth embodiment except for the operation to keep the backwardly-guided capsule medical device 2 staying at the target position. Accordingly, in the following, the operation of the capsule guiding system 61 to keep the capsule medical device 2 staying at the target position owing to the effect of the magnetic field for staying will be described with reference to FIGS. 15 and 17.

In the capsule guiding system 61 performing the backward guiding of the capsule medical device 2 in the small intestine 19 to the target position P1, the control unit 69 controls the magnetic guiding device 4 to keep the capsule medical device 2 staying at the position P1. In this case, the distance calculator 39d calculates the positional deviation quantity which is the deviation distance between the current position of the capsule medical device 2 and the position P1 based on the position information of the position P1 and the position information of the current position of the capsule medical device 2. The control unit 69 calculates the magnetic field direction of the magnetic field for staying based on the calculated positional deviation quantity and controls the magnetic guiding device 4 to generate magnetic field for staying having the calculated magnetic field direction and the previously-set magnetic field intensity.

The magnetic guiding device 4 applies, to the magnet 28 of the capsule medical device 2, the magnetic field for staying (e.g., a gradient magnetic field) as illustrated by dotted line arrows in FIG. 17 as an example based on the control of the control unit 69. The magnet 28 is moved as following the magnetic field for staying. The capsule medical device 2 incorporating the magnet 28 is magnetically guided in the direction to come closer to the position P1 with the effect of the magnetic field for staying. As a result, the capsule medical device 2 is in a staying state at the target position P1. The capsule medical device 2 backwardly moves to the target position P1 by the effect of the magnetic field for staying even when being moved by peristalsis of the small intestine 19, for example.

In the fifth embodiment of the present invention, as described above, the magnetic field for staying is applied to the capsule medical device backwardly guided to the target position in the subject to keep the capsule medical device staying at the target position by the effect of the magnetic field for staying. The rest thereof is structured similarly to the fourth embodiment. Accordingly, the functions and effects being similar to those of the above fourth embodiment are obtained. In addition, it is possible to actualize a capsule guiding system in which backward moving of a capsule medical device to a target position in a subject can be easily performed without necessity of manual operation of magnetic guiding by a user even when the capsule medical device is moved by peristalsis of an alimentary canal, for example.

### Sixth Embodiment

In the following, a sixth embodiment of the present invention will be described. In the above fifth embodiment, the capsule medical device 2 arrived at the target position by the backward guiding is kept staying at the target position with magnetic force. In the sixth embodiment, instead of keeping the capsule medical device 2 staying at the target position, the capsule medical device 2 at the target position is swung (i.e., swing operation) with a rotating magnetic field.

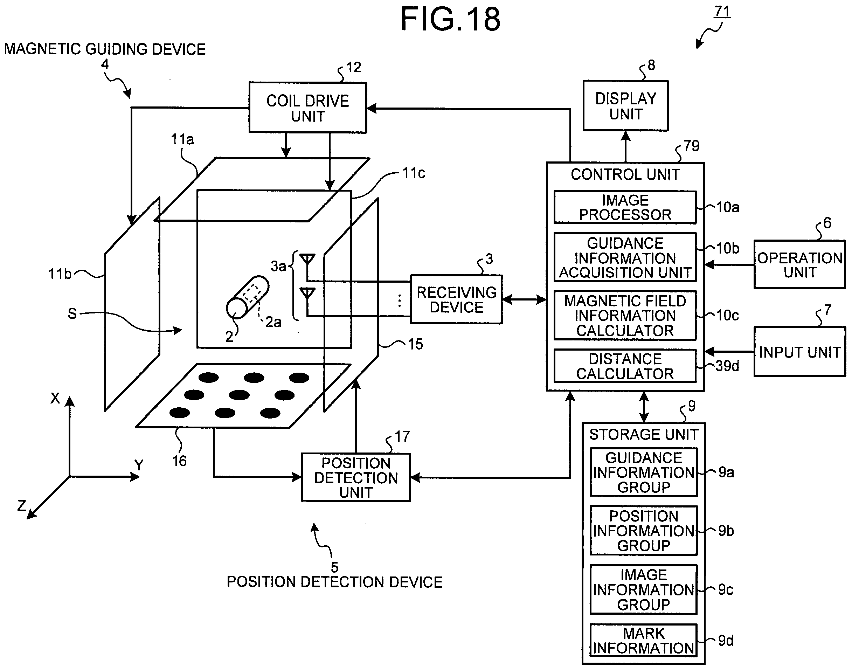

FIG. 18 is a schematic view schematically illustrating a structural example of a capsule guiding system according to the sixth embodiment of the present invention. As illustrated in FIG. 18, a capsule guiding system 71 according to the sixth embodiment is provided with a control unit 79 instead of the control unit 59 in the capsule guiding system 51 according to the above fourth embodiment. The rest of the structure is the same as the fourth embodiment and the same reference numeral is given to the same structural part.

The control unit 79 has a function to control the magnetic guiding device 4 to swing the capsule medical device 2 backwardly guided to the target position in the subject with magnetic force. Specifically, the control unit 79 obtains the current longitudinal axis direction of the capsule medical device 2 based on a direction vector etc. of the position information detected by the position detection unit 17. When the imaging unit 23 of the capsule medical device 2 is arranged in the capsule-shaped casing 21 in a state as illustrated in FIG. 2, the longitudinal axis direction of the capsule medical device 2 is matched with the imaging direction of the imaging unit 23. The control unit 79 controls the magnetic guiding device 4 to generate the rotational magnetic field so as to swing the capsule medical device 2 having the center axis of the obtained longitudinal axis direction of the capsule medical device 2 as the rotation center. The control unit 79 controls the swinging of the capsule medical device 2 at the target position in the subject through the control of the magnetic guiding device 4. Here, in addition to the function of swing control of the capsule medical device 2, the control unit 79 has similar functions to those of the control unit 59 in the capsule guiding system 51 according to the above fourth embodiment.

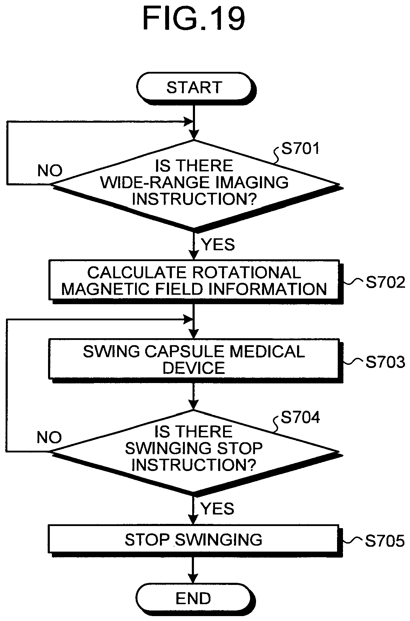

Next, the operation of the capsule guiding system 71 to swing, with magnetic force, the capsule medical device 2 backwardly guided to the target position in the subject will be described. FIG. 19 is a flowchart exemplifying operation procedure of the capsule guiding system to swing the capsule medical device with magnetic force.

For swinging the capsule medical device 2 in the subject with magnetic force, the control unit 79 in the capsule guiding system 71 firstly determines whether or not there is an instruction of wide-range imaging in the subject (step S701), as indicated in FIG. 19. Here, the wide-range imaging denotes operation to take in-vivo images with the imaging unit 23 while rotating the capsule medical device 2 about the center axis in the longitudinal axis direction of the capsule-shaped casing 21. In step S701, when instruction information of the wide-range imaging is input by the input unit 7, the control unit 79 determines that there is a wide-range imaging instruction ("Yes" in step S701). Then, the control unit 79 calculates magnetic field information for the rotational magnetic field causing the capsule medical device 2 to perform swinging required for the wide-range imaging (step S702).

In step S702, the control unit 79 obtains the current longitudinal axis direction of the capsule medical device 2 based on the direction vector etc. of the position information detected by the position detection unit 17. Then, the control unit 79 calculates a magnetic field pattern (i.e., an electric current pattern) for the rotational magnetic field so as to form a cone having a specified angle as the center axis in the obtained longitudinal axis direction being the center thereof.

Subsequently, the control unit 79 controls the magnetic guiding device 4 to swing the capsule medical device 2 with the rotational magnetic field based on the rotational magnetic field information calculated in step S702 (step S703). In step S703, the control unit 79 controls the magnetic guiding device 4 to generate the rotational magnetic field which has the calculated magnetic field pattern. In this case, the magnetic guiding device 4 applies the rotational magnetic field based on the control of the control unit 79 to the capsule medical device 2, thereby swinging the capsule medical device 2 with the rotational magnetic field.

Next, the control unit 79 determines whether or not there is an instruction to stop swinging of the capsule medical device 2 (step S704). In step S704, when instruction information to stop swinging is input by the input unit 7, the control unit 79 determines that there is an instruction to stop swinging ("Yes" in step S704). Then, the control unit 79 controls the magnetic guiding device 4 to stop swinging of the capsule medical device 2 (step S705) and the present process ends.

In step S705, the control unit 79 controls the magnetic guiding device 4 to stop generating of the rotational magnetic field to swing the capsule medical device 2. The magnetic guiding device 4 stops generating of the rotational magnetic field for the swinging based on the control of the control unit 79, thereby stopping the swinging of the capsule medical device 2.

On the contrary, in step S704, when the instruction information to stop swinging is not input, the control unit 79 determines that there is no instruction to stop swinging ("No" in step S704). Then, the control unit 79 repeats the operation procedure of step S703 and thereafter as returning to step S703.

In step S701, when the instruction information of the wide-range imaging is not input, the control unit 79 determines that there is no wide-range imaging instruction ("No" in step S701). Then, the control unit 79 repeats step S701 until the instruction information of wide-range imaging is input by the input unit 7.

Here, the control unit 79 appropriately repeats the operation procedure of steps S501 to S514 in FIG. 13 during backwardly guiding the capsule medical device 2 in the subject to the target position similarly to the control unit 59 in the capsule guiding system 51 according to the above fourth embodiment. The control unit 79 may perform the operation procedure of step S701 and thereafter subsequently to step S514 described above. In this case, after performing step S705, the control unit 79 repeats the operation procedure of step S501 and thereafter as returning to step S501 in FIG. 13. Here, the control unit 79 may perform the operation procedure of steps S701 to S705 at desired timing after the capsule medical device 2 is backwardly guided to the target position.

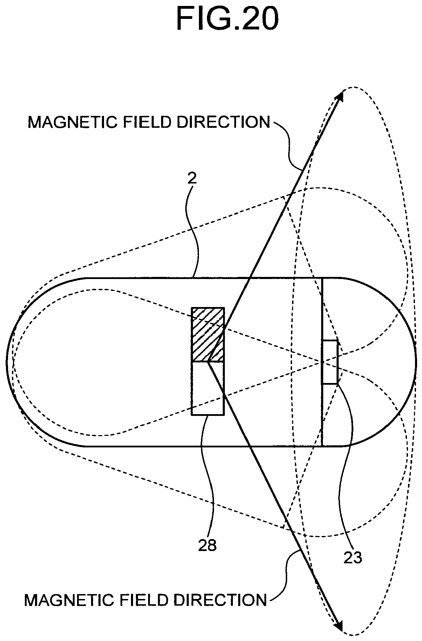

In the following, the operation of the capsule guiding system 71 according to the sixth embodiment will be specifically described. FIG. 20 is a schematic view exemplifying a state that the capsule medical device is swung at the target position in the subject. Here, the operation of the capsule guiding system 71 according to the sixth embodiment is similar to that of the capsule guiding system 51 according to the fourth embodiment except for the operation for swinging the capsule medical device 2 backwardly guided to the target position. Accordingly, in the following, the operation of the capsule guiding system 71 for swinging the capsule medical device 2 at the target position of the backward guiding is described with reference to FIGS. 18 and 20.

In the capsule guiding system 71 in which backward guiding of the capsule medical device 2 is completed to the target position in the subject (e.g., the target position P1 in the small intestine 19 and the like), the control unit 79 controls the magnetic guiding device 4 to swing the capsule medical device 2. In this case, the control unit 79 obtains the current longitudinal axis direction of the capsule medical device 2 at the target position based on the position information detected by the position detection unit 17. Then, the control unit 79 calculates the magnetic field pattern for the rotational magnetic field required for swinging of the capsule medical device 2 based on the information of the obtained longitudinal axis direction and controls the magnetic guiding device 4 to generate the rotational magnetic field having the calculated magnetic field pattern.

As illustrated in FIG. 20, the magnetic guiding device 4 generates the cone-shaped rotational magnetic field having a specified angle as the longitudinal axis of the capsule medical device 2 being the rotation center thereof. The magnet 28 of the capsule medical device 2 to which the rotational magnetic field is applied moves as following the rotational magnetic field. As a result, the capsule medical device 2 is swung along a cone as the center axis in the imaging direction of the imaging unit 23 being the rotation center, as illustrated in FIG. 20. The capsule medical device 2 in a swinging state takes in-vivo images over a wide range with the imaging unit 23 while changing the imaging direction at the inside of the subject. The in-vivo image group sequentially taken by the capsule medical device 2 which is being swung is an image group capturing the target position in the subject as well as the circumferential site thereof over a wide range to be displayed on the display unit 8. The user can observe circumference of a desired target site in the subject over a wide range and can easily locate and observe once again the in-vivo site observed with a previous in-vivo image by observing the in-vivo image group displayed on the display unit 8.

In the sixth embodiment of the present invention, as described above, the rotational magnetic field is applied to the capsule medical device backwardly guided to the target position in the subject and the capsule medical device is swung with the effect of the rotational magnetic field. The rest thereof is structured similarly to the fourth embodiment. Accordingly, the functions and effects being similar to the above fourth embodiment are obtained. In addition, it is possible to actualize a capsule guiding system in which the capsule medical device in the swinging state can take in-vivo image group at the target position and the circumference thereof over a wide range, thereby enabling to easily observe once again an in-vivo site observed with a previous in-vivo image while enabling to observe sites in the subject including the desired target position and the circumferential site thereof at the inside of the subject over a wide range.

### Seventh Embodiment

In the following, a seventh embodiment of the present invention will be described. In the above fifth embodiment, the imaging position of the in-vivo image to which the mark information 9d is assigned is set to be the target position for the backward guiding. In the seventh embodiment, one or more in-vivo images capturing a previously-set characteristic site such as a bleeding site and an affected site is automatically extracted and the mark information 9d is assigned to a desired in-vivo image selected from among the one or more automatically-extracted in-vivo images, and then, the imaging position of the in-vivo image to which the mark information 9d is assigned can be set to the target position for the backward guiding.

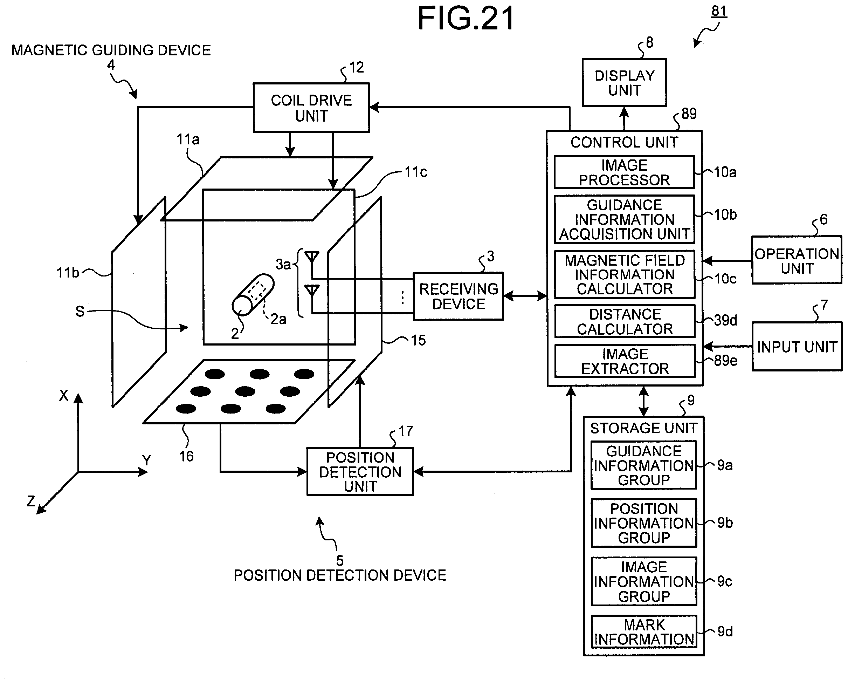

FIG. 21 is a schematic view schematically illustrating a structural example of a capsule guiding system according to the seventh embodiment of the present invention. As illustrated in FIG. 21, a capsule guiding system 81 according to the seventh embodiment is provided with a control unit 89 instead of the control unit 69 in the capsule guiding system 61 according to the above fifth embodiment. The rest of the structure is the same as the fifth embodiment and the same reference numeral is given to the same structural part.

The control unit 89 includes the image processor 10a, the guidance information acquisition unit 10b, the magnetic field information calculator 10c and the distance calculator 39d. In addition, the control unit 89 includes an image extractor 89e. Information of a characteristic site in the subject such as a bleeding site or an affected site is previously set and the image extractor 89e extracts one or more in-vivo images capturing the set characteristic site from among the in-vivo image group taken by the capsule medical device 2. In this case, the image extractor 89e extracts the in-vivo images of the characteristic site based on color information, luminance information or the like of the in-vivo images taken by the capsule medical device 2. Hereinafter, the in-vivo image capturing the characteristic site may be called as a characteristic image. The control unit 89 causes the display unit 8 to display one or more characteristic images extracted by the image extractor 89e. When the mark information 9d is input by the input unit 7, the control unit 89 assigns the mark information 9d to a desired characteristic image selected from among the one or more characteristic images with the input operation of the input unit 7 by the user. The control unit 89 sets the imaging position of the characteristic image to which the mark information 9d as described above is assigned as the target position for the backward guiding. On the contrary, when the image extractor 89e does not extract the characteristic image, the control unit 89 sets the imaging position of the in-vivo image to which the mark information 9d is assigned as the target position of the backward guiding similarly to the fifth embodiment. Here, other functions of the control unit 89 are similar to those of the control unit 69 in the capsule guiding system 61 according to the above fifth embodiment.

Here, in the seventh embodiment, the input unit 7 inputs the mark information 9d to be assigned to the characteristic image being currently-displayed image to the control unit 89 in response to predetermined input operation, such as clicking while locating a cursor at the one or more characteristic images currently displayed on the display unit 8.

When the capsule medical device 2 in the subject is backwardly guided, the control unit 89 appropriately repeats the operation procedure which is approximately similar to steps S601 to S611 in FIG. 16. That is, in step S601, the control unit 89 performs the target position setting process to set the imaging position of the characteristic image to which the mark information 9d is assigned as the target position for the backward guiding. Then, the control unit 89 performs the operation procedure of step S602 and thereafter.

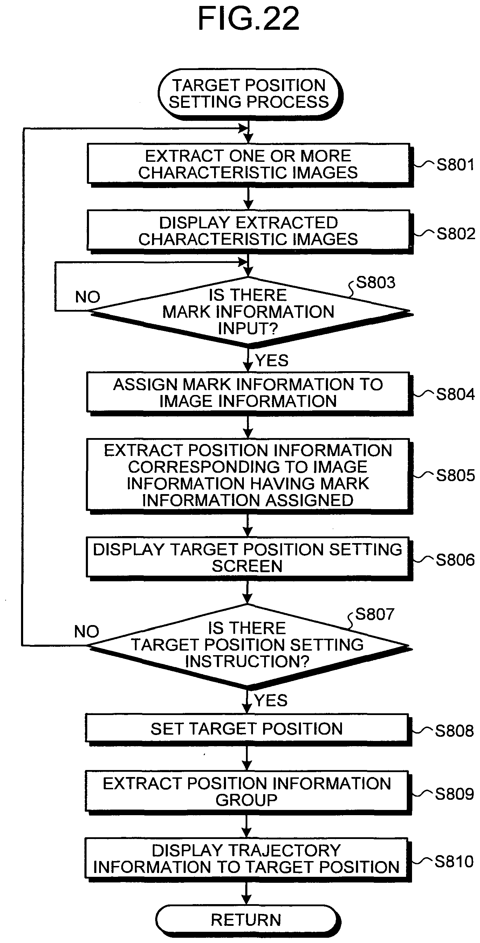

Next, the target position setting process of the seventh embodiment will be described. FIG. 22 is a flowchart exemplifying operation procedure until the control unit of the capsule guiding system according to the seventh embodiment achieves the target position setting process.

When the capsule guiding system 81 performs the target position setting process in the operation procedure of step S601 in FIG. 16, the control unit 89 firstly extracts one or more characteristic images from among the in-vivo image group taken by the capsule medical device 2 (step S801) as indicated in FIG. 22. In this case, the image extractor 89e reads out the in-vivo images from the image information group 9c in the storage unit 9 in chronological order or reverse chronological order. The image extractor 89e determines whether or not the in-vivo image is a characteristic image for each time based on color information or luminance information etc. of the read in-vivo image. The image extractor 89e extracts one or more characteristic images from among the image information group 9c by sequentially repeating the image determination process for each in-vivo image.

Next, the control unit 89 controls the display unit 8 to display the one or more characteristic images extracted in step S801 (step S802). When the image extractor 89e does not extract a characteristic image in step S801, the control unit 89 causes the display unit 8 to display information indicating that any characteristic image is not included in the image information group 9c in step S802 and controls the display unit 8 to sequentially display the respective in-vivo images in the image information group 9c in chronological order or reverse chronological order.

Subsequently, the control unit 89 determines whether or not there is input of the mark information 9d to be assigned to the characteristic image displayed on the display unit 8 (step S803). In step S803, when the mark information 9d is input by the input unit 7, the control unit 89 determines that there is input of the mark information 9d ("Yes" in step S803) and assigns the acquired mark information 9d to the image information of the characteristic image (step S804).

In step S804, the control unit 89 assigns the mark information 9d to the image information of the characteristic image which is selected by the input operation of the input unit 7 from among the one or more characteristic images currently displayed on the display unit 8. The control unit 89 controls the storage unit 9 to store the mark information 9d in association with the image information of the characteristic image which is the current in-vivo image while controlling the display unit 8 to display the mark information 9d as being superimposed with the selected characteristic image.

Next, the control unit 89 extracts position information corresponding to the image information of the characteristic image to which the mark information 9d is assigned in step S804 (step S805). In this case, the control unit 89 extracts the position information being associated with the image information of the characteristic image to which the mark information 9d is assigned from among the position information group 9b in the storage unit 9.

Then, the control unit 89 controls the display unit 8 to display a setting screen of the target position of the capsule medical device 2 for the backward guiding (step S806). In step S806, the control unit 89 causes the display unit 8 to display the setting screen inquiring with the user whether or not the imaging position of the characteristic image to which the mark information 9d is assigned in step S803 is set to be the target position of the capsule medical device 2 for the backward guiding. In this case, similarly to the case of the above second embodiment, the display unit 8 displays the setting screen on which setting or non-setting of the target position can be selected for the user with the operation of the input unit 7.

Subsequently, similarly to step S405 in FIG. 10, the control unit 89 determines whether or not there is a setting instruction of the target position of the capsule medical device 2 for the backward guiding (step S807). When it is determined that there is the setting instruction of the target position ("Yes" in step S807), the control unit 89 sets the target position of the capsule medical device 2 for the backward guiding (step S808). In step S808, the control unit 89 sets the imaging position of the characteristic image to which the mark information 9d is assigned in step S803, that is, the position information extracted in step S805 as the target position of the capsule medical device 2 during the backward guiding.

Next, the control unit 89 extracts the position information group corresponding to the movement trajectory from the target position set in step S808 to the current position of the capsule medical device 2 from among the position information group 9b in the storage unit 9 (step S809). Subsequently, the control unit 89 controls the display unit 8 to display the trajectory information from the current position to the target position of the capsule medical device 2 based on the position information group extracted in step S809 (step S810), and then, returns to step S601 in FIG. 16.

In step S810, the control unit 89 highlights the trajectory information formed by the position information group extracted in step S809, that is, the trajectory information of a section from the current position to the target position of the capsule medical device 2 with flashing or display color changing etc. from among the trajectory information of the capsule medical device 2 displayed on the display unit 8.

When the mark information 9d is not acquired from the input unit 7 in step S803, the control unit 89 determines that there is no input of the mark information 9d ("No" in step S803) and repeats step S803. Further, when it is determined that there is no setting instruction of the target position in step S807 ("No" in step S807), the control unit 89 deletes the currently-acquired position designation information, and then, the operation procedure of step S801 and thereafter is repeated as returning to step S801 thereafter.

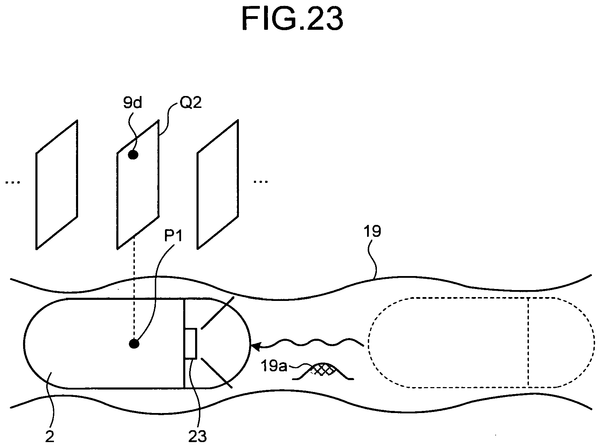

In the following, the operation of the capsule guiding system 81 according to the seventh embodiment will be specifically described with an example in which the capsule medical device 2 located at the inside of a small intestine of the subject is backwardly guided to the target position. FIG. 23 is a schematic view exemplifying a state that the capsule medical device is backwardly guided to the target position which is the imaging position of the characteristic image to which the mark information is assigned. Here, the operation of the capsule guiding system 81 according to the seventh embodiment is similar to that of the capsule guiding system 61 according to the fifth embodiment except for the operation to set the imaging position of the characteristic image to which the mark information 9d is assigned as the target position. Accordingly, in the following, the operation of the capsule guiding system 81 to set the imaging position of the characteristic image Q2 to which the mark information 9d is assigned as the target position during the backward guiding will be described with reference to FIGS. 21 and 23.

When the characteristic image Q2 capturing an affected site 19a in the small intestine 19 is included in the in-vivo image group taken by the capsule medical device 2, the control unit 89 in the capsule guiding system 81 sets the imaging position of the characteristic image Q2 as the target position for the backward guiding. In this case, the image extractor 89e extracts the characteristic image Q2 from among the in-vivo image group. The control unit 89 controls the display unit 8 to display the characteristic image Q2, and then, the control unit 89 assigns the mark information 9d to the characteristic image Q2 being the currently-displayed image when the mark information 9d is input by the input unit 7. Subsequently, the control unit 89 extracts the position information which is associated with the image information of the characteristic image Q2 to which the mark information 9d is assigned, that is, the position information of the imaging position of the characteristic image Q2, from among the position information group 9b in the storage unit 9. Then, the control unit 89 sets the position P1 corresponding to the extracted position information as the target position.

Subsequently, when the backward moving instruction information is input by the input unit 7, the control unit 89 controls the magnetic guiding device 4 to backwardly guides the capsule medical device 2 to the target position P1 as repeating the processes similar to those in the above fifth embodiment. Similarly to the case of the above fifth embodiment, the capsule medical device 2 backwardly guided by the magnetic guiding device 4 arrives at the target position P1 as backwardly moving on the own movement trajectory.

The target position P1 is approximately matched with the imaging position of the characteristic image Q2 to which the mark information 9d is assigned. The capsule medical device 2 arrived at the position P1 takes a characteristic image that is similar to the characteristic image Q2 with the imaging unit 23 in a state that the imaging field of the imaging unit 23 is directed to the direction being approximately the same as that when the characteristic image Q2 was taken. The characteristic image taken as described above by the capsule medical device 2 at the position P1 is displayed on the display unit 8. The user can easily observe once again the affected site 19a drawn in the characteristic image Q2 to which the mark information 9d is assigned by observing the characteristic image displayed on the display unit 8 as described above.

In the seventh embodiment of the present invention, as described above, one or more characteristic images are automatically extracted from among the in-vivo image group of the inside of the subject taken by the capsule medical device, and then, the imaging position of the characteristic image to which the mark information is assigned among the one or more automatically-extracted characteristic images is set as the target position for the backward guiding. The rest thereof is structured similarly to the fifth embodiment. Accordingly, the functions and effects being similar to the above fifth embodiment are obtained. In addition, it is possible to actualize a capsule guiding system in which an imaging position of a characteristic image capturing a characteristic site such as a bleeding site and an affected site can be easily set as a target position to backwardly guide a capsule medical device to the set target position without increasing operational burden for the user, thereby enabling the system to assist another observation of the characteristic site more easily.

In the above first to seventh embodiments, all of the guidance information of the magnetic guiding device 4 during the capsule medical device 2 in the subject is magnetically guided by the magnetic guiding device 4 is stored in the storage unit 9. However, the present invention is not limited to the above. Specifically, in the capsule guiding system according to each of the first to seventh embodiments, it is also possible to store and update the guidance information group of a predetermined period, which is from predetermined preceding time from the current time to the current time, in the storage unit 9 or to store and update the guidance information group of a predetermined amount of information in the storage unit 9. Further, in the capsule guiding system 31 according to the capsule guiding system 2 of the second embodiment, it is also possible to store the guidance information group corresponding to each of position information from the position on the movement trajectory designated as the target position for the backward guiding to the current position in the storage unit 9. Meanwhile, in the capsule guiding system according to each of the third to seventh embodiments, it is also possible to store, in the storage unit 9, the guidance information group over a period from the mark information 9d is input to the current time, that is, the guidance information group of the magnetic guiding device 4 during magnetically guiding the capsule medical device 2 from the past position being the imaging position of the in-vivo image to which the mark information 9d is assigned to the current position.

Further, in the above third to seventh embodiments, each of guidance information in the guidance information group 9a of the magnetic guiding device 4, each of position information in the position information group 9b of the capsule medical device 2, and each of in-vivo images in the image information group 9c of the subject are respectively associated in chronological order. However, not limited to the above, each of guidance information in the guidance information group 9a may not be associated with each of position information in the position information group 9b as being only required to be associated at least with each of in-vivo images in the image information group 9c in chronological order. In this case, the guidance information group necessary for calculating the magnetic field information for the backward guiding of the capsule medical device 2 is only required to be sequentially extracted from the storage unit 9 in reverse chronological order as the guidance information group being associated with the in-vivo image group sequentially taken at each position on the movement trajectory on which the capsule medical device 2 is to be backwardly moved.

Further, in the above first to seventh embodiments, the capsule medical device 2 in the subject is backwardly guided automatically from the current position to the target position when the backward moving instruction information is input by the input unit 7. In the capsule guiding system according to each of the first to seventh embodiment, it is also possible that the capsule medical device 2 is magnetically guided to move frontward along an alimentary canal from the target position to the original position (i.e., the current position before the backward guiding) based on the magnetic field information of the magnetic guiding device 4 for backwardly moving to the target position (i.e., the guidance information during the backward guiding). In this case, in the capsule guiding system, the control unit sequentially acquires guidance information of the magnetic guiding device 4 in chronological order during backwardly guiding the capsule medical device 2 and the storage unit sequentially stores the guidance information during the backward guiding acquired by the control unit in chronological order. When the frontward-moving instruction information to instruct frontward moving of the capsule medical device 2 is input by the input unit 7, the control unit is only required to control the magnetic guiding device 4 so that the capsule medical device 2 in the subject moves frontward to the original position on the own movement trajectory based on the guidance information group during the backward guiding stored in the storage unit.

Further, in the above first to seventh embodiment, the display unit 8 displays the information indicating that the capsule medical device 2 has arrived at the target position for the backward guiding or the information indicating that the backward guiding of the capsule medical device 2 has stopped. However, not limited to the above, such information may be output to the outside as audio information such as beeps.

Further, in the above first to seventh embodiment, the display unit 8 displays current trajectory information indicating the movement trajectory of the capsule medical device 2 introduced into the subject this time. However, not limited to the above, the display unit 8 may further display past trajectory information indicating the movement trajectory of the capsule medical device introduced into the subject in the past in addition to the current trajectory information. In this case, the current trajectory information and the past trajectory information may be displayed on the display unit 8 as being superimposed in mutually different display forms such as colors or shapes. In the capsule guiding system having the display unit 8, when designation information of a positional point on the past trajectory information is input as the target position for the backward guiding, the control unit may control the magnetic guiding device 4 so that the capsule medical device 2 backwardly moves on the past trajectory information while setting the position information on the current trajectory information being closest to the designated past position information as the target position.

Further, in the above first to seventh embodiments, the capsule medical device 2 is backwardly moved to the desired in-vivo position in the subject with the backward guiding. Not limited to the above, it is also possible to add additional information such as a note or a comment to the imaging position (i.e., observation position) of an in-vivo image when the capsule medical device 2 arrived at the desired in-vivo position in the subject newly takes an in-vivo image and to store the additional information and the guidance information in the storage unit 9 in association with each other. In this case, the input unit 7 further inputs the additional information such as a note or a comment to be added to the in-vivo image displayed on the display unit 8. The additional information input by the input unit 7 and the in-vivo image taken by the capsule medical device 2 at the desired in-vivo position are stored in the storage unit 9 in association with each other. Accordingly, when a keyword related to the additional information is input by the input unit 7, the control unit can easily set the imaging position of the in-vivo image associated with the additional information including the keyword as the target position, thereby enabling a user to easily set the target position of the capsule medical device 2.

Further, in the above first to seventh embodiments, various types of information is stored in the storage unit 9 in association with an in-vivo image. Not limited to the above, the information to be stored in the storage unit 9 may include information indicating a situation of magnetic guiding of the capsule medical device 2, for example, in addition to the above. Specifically, in a case of having difficulty for the capsule medical device 2 in the subject to pass through an alimentary canal during guiding operation of the capsule medical device 2, magnetic attractive force or torque to be generated for the capsule medical device 2 becomes large. In this case, a reference value of the magnetic attractive force or torque is stored in the storage unit 9. The control unit 10 compares magnitude of the generated magnetic attractive force or torque with the reference value. If the magnitude of the magnetic attractive force or torque exceeds the reference value, the image taken by the capsule medical device 2 when generating the magnetic field exceeding the reference value and information of the magnetic attractive force or torque (i.e., information indicating a situation of magnetic guiding of the capsule medical device 2) are stored in the storage unit 9 in association with each other. In this case, the storage unit 9 stores the information of the magnetic attractive force or torque as the mark information. It becomes possible to backwardly guide the capsule medical device 2 for each section of guiding operation by setting a target point for the backward guiding of the capsule medical device 2 by utilizing the mark information.

Meanwhile, movement speed and a stability situation of the capsule medical device 2 in the subject can be recognized by utilizing the position detection result of the capsule medical device 2 detected by the position detection unit 17. Specifically, a threshold value regarding movement quantity per unit time is set and the set threshold value is previously stored in the storage unit 9 as the mark information to be associated with the in-vivo image. The control unit 10 can determine whether or not the capsule medical device 2 in the subject is in a moving state (i.e., a non-stopped state) by comparing the threshold value with movement quantity based on the position information of the capsule medical device 2. In addition, a threshold value regarding movement speed of the capsule medical device 2 is further set and the set threshold value is previously stored in the storage unit 9 as the mark information to be associated with the in-vivo image. The control unit 10 can determine whether or not the capsule medical device is moved smoothly within the subject by comparing the threshold value with the movement speed of the capsule medical device 2.

In this case, the mark information regarding the movement speed can be acquired as "the capsule medical device 2 stays", "the capsule medical device 2 moves slowly", " the capsule medical device 2 moves quickly", or the like, for example. The mark information regarding the movement speed and the acquired image (i.e., the in-vivo image) of the capsule medical device 2 are stored in the storage unit 9 in association with each other.

Actual marking on the in-vivo image requires operation to select a marking objective image from among plural in-vivo images based on the information related to the desired in-vivo image and the guiding situation of the capsule medical device 2. When the number of the all images is enormous, the operation itself becomes difficult. In order to avoid the above, it is also possible to reduce the number of selecting objective images by previously excluding in-vivo images taken while the capsule medical device 2 is in a stopped state, for example. Further, in order to perform observation once again for checking if there is missing of an in-vivo site through which once passing rapidly, it is also possible to adopt a method of extracting only the in-vivo images during rapid moving of the capsule medical device 2. The method similarly has the effect to reduce the number of the selecting objective images as well.

Furthermore, it is also possible to perform marking of special operation regarding the guiding. Here, the special operation denotes operation as being previously programmed for an observation purpose. More specifically, the special operation denotes operation capable of being specified by a variation pattern of the magnetic field generated by the magnetic field generation units 11a to 11c varied with voltage output by the coil drive unit 12 in FIG. 1. For controlling the magnetic guiding of the capsule medical device 2, the control unit of the capsule guiding system causes the magnetic field generation units 11a to 11c to generate the variation pattern of the magnetic field in a state that the special operation is performed. Accordingly, the generation instruction thereof can be utilized as a trigger. The same result can be obtained by directly detecting the pattern of the voltage or the magnetic field described above.

As the operation of the capsule medical device 2 based on the special operation, there may be exemplified swing operation of the capsule medical device 2 described in the above sixth embodiment, imaging operation of in-vivo images while the capsule medical device 2 is moved underwater at a gastric region etc. in a spiral, or the like. In any of the operation, the information indicating that the special operation to cause operation of the capsule medical device 2 occurs during the guiding control (i.e., the information related to the variation pattern of the magnetic field generated by the magnetic field generation units 11a to 11c) is stored in the storage unit 9 as the mark information in association with the in-vivo image. It becomes possible to observe once again an in-vivo site at a certain position as backwardly moves the capsule medical device 2 to the position later by utilizing the information stored in the storage unit 9.

Any of the above marking can be directly utilized as the target point of the backward guiding of the capsule medical device 2. In addition, it is possible to obtain an effect of easy refinement as reducing the number of observation objective images even when the acquired in-vivo images are simply to be read later.

Meanwhile, in the above sixth embodiment, the control unit 79 controls the rotational magnetic field of the magnetic guiding device 4 to swing the capsule medical device 2 until the instruction information to stop swinging of the capsule medical device 2 is input. However, not limited to the above, it is also possible that the control unit 79 controls the magnetic guiding device 4 to stop the capsule medical device 2 with magnetic force at the target position similarly to the above fifth embodiment when the capsule medical device 2 has swung by a predetermined number of swings (e.g., one swing).

Further, in the above first to seventh embodiments, the capsule guiding system to magnetically guide the capsule medical device 2 incorporating the imaging function and the wireless communication function is described as an example. The capsule medical device magnetically guided in the capsule guiding system according to the present invention may be a capsule pH measurement device to measure pH in a living body, a capsule medication device having a function to spray or inject medicine in a living body, or a capsule harvest device to harvest material in a living body, as long as incorporating the imaging function and the wireless communication function.

### Industrial Applicability

As described above, the capsule guiding system according to the present invention is useful for magnetic guiding of a capsule medical device introduced into a subject, and in particular, is suitable for a capsule guiding system capable of reducing operational burden for a user to backwardly move the capsule medical device in the subject.

### Reference Signs List

- 1, 31, 41, 51, 61, 71, 81: CAPSULE GUIDING SYSTEM
- 2: CAPSULE MEDICAL DEVICE
- 2a: LC MARKER
- 3: RECEIVING DEVICE
- 3a: RECEIVING ANTENNA
- 4: MAGNETIC GUIDING DEVICE
- 5: POSITION DETECTION DEVICE
- 6: OPERATION UNIT
- 7: INPUT UNIT
- 8: DISPLAY UNIT
- 9: STORAGE UNIT
- 9a: GUIDANCE INFORMATION GROUP
- 9b: POSITION INFORMATION GROUP
- 9c: IMAGE INFORMATION GROUP
- 9d: MARK INFORMATION
- 10, 39, 49, 59, 69, 79, 89: CONTROL UNIT
- 10a: IMAGE PROCESSOR
- 10b: GUIDANCE INFORMATION ACQUISITION UNIT
- 10c: MAGNETIC FIELD INFORMATION CALCULATOR
- 11a to 11c, 15: MAGNETIC FIELD GENERATION UNIT
- 12: COIL DRIVE UNIT
- 16: MAGNETIC FIELD DETECTION UNIT
- 17: POSITION DETECTION UNIT
- 19: SMALL INTESTINE
- 21: CAPSULE-SHAPED CASING
- 21a: CYLINDRICAL CASING
- 21b: DOME-SHAPED CASING
- 22: ILLUMINATION UNIT
- 23: IMAGING UNIT
- 23a: CONDENSER LENS
- 23b: SOLID-STATE IMAGING ELEMENT
- 24: SIGNAL PROCESSOR
- 25: TRANSMITTER
- 26: CONTROLLER
- 27: BATTERY
- 28: MAGNET
- 39d: DISTANCE CALCULATOR
- 89e: IMAGE EXTRACTOR
- F: MOVEMENT TRAJECTORY
- Q1: IN-VIVO IMAGE
- Q2: CHARACTERISTIC IMAGE
- S: THREE-DIMENSIONAL SPACE

## Claims

1. A capsule guiding system (41; 51; 61; 71; 81) comprising:
a capsule medical device (2) that is introducible into a subject and configured to take an in-vivo image in the subject;
a capsule guiding unit (4) configured to guide the capsule medical device;
an image acquisition unit configured to acquire the in-vivo image of the subject from the capsule medical device;
a display unit (8) configured to display the in-vivo image of the subject acquired by the image acquisition unit;
a storage unit (9) configured to sequentially store guidance information of the capsule guiding unit (4) that is configured to guide the capsule medical device (2), **characterized in that** the capsule guiding system comprises:
an input unit (7) configured to input instruction information to instruct start of a backward moving of the capsule medical device (2), and configured to input mark information (9d) to be assigned to the in-vivo image displayed on the display unit (8) as position information relating to a target position where the backward moving is stopped; and
a control unit (49; 59; 69; 79; 89) configured to control, when the instruction information instructing start of the backward moving is input, the capsule guiding unit (4) such that the capsule medical device (2) backwardly moves on a trajectory based on the guidance information of the capsule guiding unit (4) stored in the storage unit (9), and configured to control the capsule guiding unit (4) such that the capsule medical device (2) stops the backward moving based on the position information relating to a target position where the backward moving is stopped.

2. The capsule guiding system (41) according to claim 1, wherein
the storage unit (9) is configured to sequentially store guidance information of the capsule guiding unit (4) for guiding the capsule medical device (2) from a certain past position being an imaging position of the in-vivo image and the target position where the backward moving is stopped to which the mark information (9d) is assigned to a current position at which the capsule medical device (2) currently exists, and
the control unit (49) is configured to control the capsule guiding unit (4) to guide the capsule medical device (2) from the current position to the past position.

3. The capsule guiding system (41) according to claim 2,
wherein the storage unit (9) is configured to sequentially store an in-vivo image taken by the capsule medical device (2) in association with guidance information of the capsule guiding unit (4), and
the control unit (49) is configured to control the capsule guiding unit to guide the capsule medical device to an imaging position of a past in-vivo image relative to an imaging position of an in-vivo image currently displayed on the display unit (8).

4. The capsule guiding system (41) according to claim 1, wherein
the storage unit (9) is configured to sequentially store an in-vivo image taken by the capsule medical device (2) in association with guidance information of the capsule guiding unit (4), and
the control unit (49) is configured to extract, from the storage unit, guidance information of the capsule guiding unit for guiding the capsule medical device from a past position being an imaging position of the in-vivo image to which the mark information is assigned to a current position and is configured to control the capsule guiding unit to guide the capsule medical device from the current position to the past position based on the extracted guidance information.

5. The capsule guiding system (51) according to claim 2,
wherein the control unit is configured to determine whether or not the capsule medical device (2) has arrived at a vicinity of the past position being targeted and is configured to control the capsule guiding unit (4) to reduce guiding speed of the capsule medical device (2) to low speed after arriving at the vicinity of the past position compared to that before arriving thereat.

6. The capsule guiding system (61) according to claim 2,
wherein the control unit (69) is configured to determine whether or not the capsule medical device (2) has arrived at the past position being targeted and is configured to control the capsule guiding unit (4) to keep the capsule medical device (2) staying at the arrived past position if the capsule medical device (2) has arrived at the past position.

7. The capsule guiding system (71) according to claim 2,
wherein the control unit (79) is configured to determine whether or not the capsule medical device (2) has arrived at the past position being targeted and is configured to control the capsule guiding unit (4) to swing the capsule medical device (2) if the capsule medical device (2) has arrived at the past position.

8. The capsule guiding system (41; 51; 61; 71; 81) according to claim 1,
wherein the input unit (7) is configured to input frontward-moving instruction information to instruct frontward moving of the capsule medical device (2), and
the control unit (49; 59; 69; 79; 89) is configured to control the capsule guiding unit (4) to cause the capsule medical device (2) to move frontward on a trajectory based on the guidance information of the capsule guiding unit (4) stored in the storage unit when the frontward instruction information is input.

9. The capsule guiding system (81) according to claim 3,
wherein the control unit (89) is configured to extract at least one or more characteristic images having predetermined character from among an in-vivo image group of the subject taken by the capsule medical device (2) and is configured to control the display unit (8) to display the extracted characteristic images, and
the input unit (7) is configured to input the mark information (9d) to be assigned to any of the characteristic images displayed on the display unit (8).

10. The capsule guiding system (41; 51; 61; 71; 81) according to claim 2,
wherein the input unit (7) is further configured to input additional information to be added to the in-vivo image displayed on the display unit (8), and
the storage unit (9) is configured to store the in-vivo image, the additional information and the guidance information of the capsule guiding unit (4) in association with each other.

11. The capsule guiding system (41; 51; 61; 71; 81) according to claim 1,
wherein the mark information is information related to attractive force exerted on the capsule medical device by the capsule guiding unit.

12. The capsule guiding system (41; 51; 61; 71; 81) according to claim 1,
wherein the mark information is information related to torque exerted on the capsule medical device by the capsule guiding unit.

13. The capsule guiding system (41; 51; 61; 71; 81) according to claim 1,
wherein the mark information is information related to movement speed of the capsule medical device.

14. The capsule guiding system (41; 51; 61; 71; 81) according to claim 1,
wherein the mark information is information related to a previously-defined variation pattern of a magnetic field to be generated by the capsule guiding unit.

## Patentansprüche

1. Kapselführungssystem (41; 51; 61; 71; 81), umfassend:
eine medizinische Kapselvorrichtung (2), die in ein Subjekt einführbar ist und dazu eingerichtet ist, um in dem Subjekt ein in-vivo Bild aufzunehmen;
eine Kapselführungseinheit (4), die dazu eingerichtet ist, um die medizinische Kapselvorrichtung zu führen;
eine Bildbezugseinheit, die dazu eingerichtet ist, um das in-vivo Bild von dem Subjekt von der medizinischen Kapselvorrichtung zu beziehen;
eine Anzeigeeinheit (8), die dazu eingerichtet ist, um das durch die Bildbezugseinheit bezogene in-vivo Bild von dem Subjekt anzuzeigen;
eine Speichereinheit (9), die dazu eingerichtet ist, um Führungsinformation der zum Führen der medizinischen Kapselvorrichtung (2) eingerichteten Kapselführungseinheit (4) fortlaufend zu speichern, **dadurch gekennzeichnet, dass** das Kapselführungssystem umfasst:
eine Eingabeeinheit (7), die dazu eingerichtet ist, um Befehlsinformation einzugeben, um einen Start einer Rückwärtsbewegung der medizinischen Kapselvorrichtung (2) anzuordnen und die dazu eingerichtet ist, um dem auf der Anzeigeeinheit (8) angezeigten in-vivo Bild zuzuordnende Markierungsinformation (9d) als Positionsinformation in Bezug auf eine Zielposition einzugeben, an der die Rückwärtsbewegung gestoppt wird; und
eine Steuereinheit (49; 59; 69; 79; 89), die dazu eingerichtet ist, um, wenn die den Start der Rückwärtsbewegung anordnende Befehlsinformation eingegeben wird, die Kapselführungseinheit (4) derart zu steuern, dass sich die medizinische Kapselvorrichtung (2) basierend auf der in der Speichereinheit (9) gespeicherten Führungsinformation der Kapselführungseinheit (4) auf einer Trajektorie rückwärts bewegt und die dazu eingerichtet ist, um die Kapselführungseinheit (4) derart zu steuern, dass die medizinische Kapselvorrichtung (2) die Rückwärtsbewegung basierend auf der Positionsinformation in Bezug auf eine Zielposition stoppt, an der die Rückwärtsbewegung gestoppt wird.

2. Kapselführungssystem (41) nach Anspruch 1, bei dem die Speichereinheit (9) dazu eingerichtet ist, um die Führungsinformation der Kapselführungseinheit (4) fortlaufend zu speichern, um die medizinische Kapselvorrichtung (2) von einer bestimmten vergangenen Position, die eine Abbildungsposition des in-vivo Bildes ist und die die Zielposition ist, an der die Rückwärtsbewegung gestoppt wird und der die Markierungsinformation (9d) zugeordnet ist, an eine aktuelle Position zu führen, an der sich die medizinische Kapselvorrichtung (2) aktuell befindet, und
die Steuereinheit (49) dazu eingerichtet ist, um die Kapselführungseinheit (4) zu steuern, um die medizinische Kapselvorrichtung (2) von der aktuellen Position an die vergangene Position zu führen.

3. Kapselführungssystem (41) nach Anspruch 2, bei dem die Speichereinheit (9) dazu eingerichtet ist, um ein von der medizinischen Kapselvorrichtung (2) aufgenommenes in-vivo Bild fortlaufend in Verbindung mit der Führungsinformation der Kapselführungseinheit (4) zu speichern, und
die Steuereinheit (49) dazu eingerichtet ist, um die Kapselführungseinheit zu steuern, um die medizinische Kapselvorrichtung an eine Abbildungsposition eines vergangenen in-vivo Bildes relativ zu einer Abbildungsposition eines aktuell auf der Anzeigeeinheit (8) angezeigten in-vivo Bildes zu führen.

4. Kapselführungssystem (41) nach Anspruch 1, bei dem
die Speichereinheit (9) dazu eingerichtet ist, um ein von der medizinischen Kapselvorrichtung (2) aufgenommenes in-vivo Bild fortlaufend in Verbindung mit der Führungsinformation der Kapselführungseinheit (4) zu speichern, und
die Steuereinheit (49) dazu eingerichtet ist, um aus der Speichereinheit die Führungsinformation der Kapselführungseinheit zu entnehmen, um die medizinische Kapselvorrichtung von einer vergangenen Position, die eine Abbildungsposition des in-vivo Bildes ist und der die Markierungsinformation zugeordnet ist, an eine aktuelle Position zu führen und dazu eingerichtet ist, um die Kapselführungseinheit zu steuern, um die medizinische Kapselvorrichtung basierend auf der entnommenen Führungsinformation von der aktuellen Position an die vergangene Position zu führen.

5. Kapselführungssystem (51) nach Anspruch 2, bei dem die Steuereinheit dazu eingerichtet ist, um zu bestimmen, ob die medizinische Kapselvorrichtung (2) in der Nähe der vergangenen Position, die die Zielposition ist, angekommen ist oder nicht und dazu eingerichtet ist, um die Kapselführungseinheit (4) zu steuern, um eine Führungsgeschwindigkeit der medizinischen Kapselvorrichtung (2) nach dem Ankommen in der Nähe der vergangenen Position auf eine verglichen mit vor dem dortigen Ankommen niedrige Geschwindigkeit zu reduzieren.

6. Kapselführungssystem (61) nach Anspruch 2, bei dem die Steuereinheit (69) dazu eingerichtet ist, um zu bestimmen, ob die medizinische Kapselvorrichtung (2) an der vergangenen Position, die die Zielposition ist, angekommen ist oder nicht und dazu eingerichtet ist, um die Kapselführungseinheit (4) zu steuern, um ein Stehenbleiben der medizinischen Kapselvorrichtung (2) an der erreichten vergangenen Position aufrecht zu erhalten, wenn die medizinische Kapselvorrichtung (2) an der vergangenen Position angekommen ist.

7. Kapselführungssystem (71) nach Anspruch 2, bei dem die Steuereinheit (79) dazu eingerichtet ist, um zu bestimmen, ob die medizinische Kapselvorrichtung (2) an der vergangenen Position, die die Zielposition ist, angekommen ist oder nicht und dazu eingerichtet ist, um die Kapselführungseinheit (4) zu steuern, um die medizinische Kapselvorrichtung (2) zu schwenken, wenn die medizinische Kapselvorrichtung (2) an der vergangenen Position angekommen ist.

8. Kapselführungssystem (41; 51; 61; 71; 81) nach Anspruch 1, bei dem die Eingabeeinheit (7) dazu eingerichtet ist, um Vorwärtsbewegungs-Befehlsinformation einzugeben, um eine Vorwärtsbewegung der medizinischen Kapselvorrichtung (2) anzuordnen und die Steuereinheit (49; 59; 69; 79; 89) dazu eingerichtet ist, um die Kapselführungseinheit (4) zu steuern, um die medizinische Kapselvorrichtung (2) zu veranlassen, sich basierend auf der in der Speichereinheit gespeicherten Führungsinformation der Kapselführungseinheit (4) auf einer Trajektorie vorwärts zu bewegen, wenn die Vorwärts-Befehlsinformation eingegeben wird.

9. Kapselführungssystem (81) nach Anspruch 3, bei dem die Steuereinheit (89) dazu eingerichtet ist, um zumindest eines oder mehrere charakteristische Bilder mit einer vorbestimmten Eigenschaft aus einer von der medizinischen Kapselvorrichtung (2) aufgenommenen Gruppe von in-vivo Bildern des Subjekts zu entnehmen und dazu eingerichtet ist, um die Anzeigeeinheit (8) zu steuern, um die entnommenen charakteristischen Bilder anzuzeigen und die Eingabeeinheit (7) dazu eingerichtet ist, um die Markierungsinformation (9d) einzugeben, um irgendeinem der auf der Anzeigeeinheit (8) angezeigten charakteristischen Bilder zugeordnet zu werden.

10. Kapselführungssystem (41; 51; 61; 71; 81) nach Anspruch 2, bei dem die Eingabeeinheit (7) des Weiteren dazu eingerichtet ist, um Zusatzinformation einzugeben, die dem auf der Anzeigeeinheit (8) angezeigten in-vivo Bild hinzuzufügen ist, und
die Speichereinheit (9) dazu eingerichtet ist, um das in-vivo Bild, die Zusatzinformation und die Führungsinformation der Kapselführungseinheit (4) in Verbindung miteinander zu speichern.

11. Kapselführungssystem (41; 51; 61; 71; 81) nach Anspruch 1, bei dem die Markierungsinformation Information in Bezug auf eine von der Kapselführungseinheit auf die medizinische Kapselvorrichtung ausgeübte Anziehungskraft ist.

12. Kapselführungssystem (41; 51; 61; 71; 81) nach Anspruch 1, bei dem die Markierungsinformation Information in Bezug auf ein von der Kapselführungseinheit auf die medizinische Kapselvorrichtung ausgeübtes Moment ist.

13. Kapselführungssystem (41; 51; 61; 71; 81) nach Anspruch 1, bei dem die Markierungsinformation Information in Bezug auf eine Bewegungsgeschwindigkeit der medizinischen Kapselvorrichtung ist.

14. Kapselführungssystem (41; 51; 61; 71; 81) nach Anspruch 1, bei dem die Markierungsinformation Information in Bezug auf ein vorhergehend definiertes Variationsmuster eines von der Kapselführungseinheit zu erzeugenden Magnetfeldes ist.

## Revendications

1. Système (41 ; 51 ; 61 ; 71 ; 81) de guidage de capsule comprenant :
un dispositif médical de type capsule (2) qui peut être introduit dans un sujet et est configuré pour prendre une image *in vivo* dans le sujet ;
une unité (4) de guidage de capsule configurée pour guider le dispositif médical de type capsule ;
une unité d'acquisition d'image configurée pour acquérir l'image *in vivo* du sujet à partir du dispositif médical de type capsule ;
une unité (8) d'affichage configurée pour afficher l'image *in vivo* du sujet acquise par l'unité d'acquisition d'image ;
une unité (9) de stockage configurée pour stocker séquentiellement des informations de guidage de l'unité (4) de guidage de capsule qui est configurée pour guider le dispositif médical de type capsule (2),
**caractérisé en ce que** le système de guidage de capsule comprend :
une unité (7) d'entrée configurée pour entrer des informations d'instruction pour indiquer un début d'un mouvement vers l'arrière du dispositif médical de type capsule (2), et configurée pour entrer une information (9d) de marquage devant être affectée à l'image *in vivo* affichée sur l'unité (8) d'affichage comme une information de position se rapportant à une position cible où le mouvement vers l'arrière est stoppé ; et
une unité (49 ; 59 ; 69 ; 79 ; 89) de commande configurée pour commander, lorsque les informations d'instruction indiquant le début du mouvement vers l'arrière sont entrées, l'unité (4) de guidage de capsule de telle manière que le dispositif médical de type capsule (2) se déplace vers l'arrière sur une trajectoire basée sur les informations de guidage de l'unité (4) de guidage de capsule stockées dans l'unité (9) de stockage, et configurée pour commander l'unité (4) de guidage de capsule de telle manière que le dispositif médical de type capsule (2) stoppe le mouvement vers l'arrière sur la base de l'information de position se rapportant à une position cible où le mouvement vers l'arrière est stoppé.

2. Système (41) de guidage de capsule selon la revendication 1, dans lequel
l'unité (9) de stockage est configurée pour stocker séquentiellement des informations de guidage de l'unité (4) de guidage de capsule pour guider le dispositif médical de type capsule (2) d'une certaine position passée étant une position d'imagerie de l'image *in vivo* et la position cible où le mouvement vers l'arrière est stoppé et à laquelle l'information (9d) de marquage est affectée à une position courante à laquelle le dispositif médical de type capsule (2) existe couramment, et
l'unité de commande (49) est configurée pour commander l'unité (4) de guidage de capsule pour guider le dispositif médical de type capsule (2) de la position courante à la position passée.

3. Système (41) de guidage de capsule selon la revendication 2,
dans lequel l'unité (9) de stockage est configurée pour stocker séquentiellement une image *in vivo* prise par le dispositif médical de type capsule (2) en association avec des informations de guidage de l'unité (4) de guidage de capsule, et
l'unité de commande (49) est configurée pour commander l'unité de guidage de capsule pour guider le dispositif médical de type capsule jusqu'à une position d'imagerie d'une image *in vivo* passée par rapport à une position d'imagerie d'une image *in vivo* couramment affichée sur l'unité (8) d'affichage.

4. Système (41) de guidage de capsule selon la revendication 1, dans lequel
l'unité (9) de stockage est configurée pour stocker séquentiellement une image *in vivo* prise par le dispositif médical de type capsule (2) en association avec des informations de guidage de l'unité (4) de guidage de capsule, et
l'unité de commande (49) est configurée pour extraire, de l'unité de stockage, des informations de guidage de l'unité de guidage de capsule pour guider le dispositif médical de type capsule d'une position passée étant une position d'imagerie de l'image *in vivo* à laquelle l'information de marquage est affectée jusqu'à une position courante et est configurée pour commander l'unité de guidage de capsule pour guider le dispositif médical de type capsule de la position courante à la position passée sur la base des informations de guidage extraites.

5. Système (51) de guidage de capsule selon la revendication 2,
dans lequel l'unité de commande est configurée pour déterminer si le dispositif médical de type capsule (2) est ou non arrivé à proximité de la position passée étant ciblée et est configurée pour commander l'unité (4) de guidage de capsule pour réduire une vitesse de guidage du dispositif médical de type capsule (2) à une vitesse basse après l'arrivée à proximité de la position passée par rapport à celle avant l'arrivée à celle-ci.

6. Système (61) de guidage de capsule selon la revendication 2,
dans lequel l'unité (69) de commande est configurée pour déterminer si le dispositif médical de type capsule (2) est ou non arrivé à la position passée étant ciblée et est configurée pour commander l'unité (4) de guidage de capsule pour maintenir le dispositif médical de type capsule (2) présent à la position passée atteinte si le dispositif médical de type capsule (2) est arrivé à la position passée.

7. Système (71) de guidage de capsule selon la revendication 2,
dans lequel l'unité (79) de commande est configurée pour déterminer si le dispositif médical de type capsule (2) est ou non arrivé à la position passée étant ciblée et est configurée pour commander l'unité (4) de guidage de capsule pour retourner le dispositif médical de type capsule (2) si le dispositif médical de type capsule (2) est arrivé à la position passée.

8. Système (41 ; 51 ; 61 ; 71 ; 81) de guidage de capsule selon la revendication 1,
dans lequel l'unité (7) d'entrée est configurée pour entrer des informations d'instruction de mouvement vers l'avant pour indiquer un mouvement vers l'avant du dispositif médical de type capsule (2), et
l'unité (49 ; 59 ; 69 ; 79 ; 89) de commande est configurée pour commander l'unité (4) de guidage de capsule pour faire en sorte que le dispositif médical de type capsule (2) se déplace vers l'avant sur une trajectoire basée sur les informations de guidage de l'unité (4) de guidage de capsule stockées dans l'unité de stockage lorsque les informations d'instruction de mouvement vers l'avant sont entrées.

9. Système (81) de guidage de capsule selon la revendication 3,
dans lequel l'unité (89) de commande est configurée pour extraire au moins une ou plusieurs image(s) caractéristique(s) ayant un caractère prédéterminé parmi un groupe d'images *in vivo* du sujet prises par le dispositif médical de type capsule (2) et est configurée pour commander l'unité (8) d'affichage pour afficher les images caractéristiques extraites, et
l'unité (7) d'entrée est configurée pour entrer l'information (9d) de marquage devant être affectée à une quelconque des images caractéristiques affichées sur l'unité (8) d'affichage.

10. Système (41 ; 51 ; 61 ; 71 ; 81) de guidage de capsule selon la revendication 2,
dans lequel l'unité (7) d'entrée est en outre configurée pour entrer des informations additionnelles devant être ajoutées à l'image *in vivo* affichée sur l'unité (8) d'affichage, et
l'unité (9) de stockage est configurée pour stocker l'image *in vivo,* les informations additionnelles et les informations de guidage de l'unité (4) de guidage de capsule en association les unes avec les autres.

11. Système (41 ; 51 ; 61 ; 71 ; 81) de guidage de capsule selon la revendication 1,
dans lequel l'information de marquage est une information se rapportant à une force d'attraction exercée sur le dispositif médical de type capsule par l'unité de guidage de capsule.

12. Système (41 ; 51 ; 61 ; 71 ; 81) de guidage de capsule selon la revendication 1,
dans lequel l'information de marquage est une information se rapportant à un couple exercé sur le dispositif médical de type capsule par l'unité de guidage de capsule.

13. Système (41 ; 51 ; 61 ; 71 ; 81) de guidage de capsule selon la revendication 1,
dans lequel l'information de marquage est une information se rapportant à une vitesse de mouvement du dispositif médical de type capsule.

14. Système (41 ; 51 ; 61 ; 71 ; 81) de guidage de capsule selon la revendication 1,
dans lequel l'information de marquage est une information se rapportant à un motif de variation préalablement défini d'un champ magnétique devant être généré par l'unité de guidage de capsule.
